# EUROPEAN PATENT SPECIFICATION

(11) **EP 3 533 391 B1**
(45) Date of publication and mention of the grant of the patent: **02.04.2025**
(21) Application number: 17865523.9
(22) Date of filing: 28.07.2017
(51) Int. Cl.: A61B 5/026, A61B 5/0245, A61B 5/1455, A61B 5/00

(54) **INFORMATION PROCESSING DEVICE, INFORMATION PROCESSING METHOD, AND PROGRAM**
INFORMATIONSVERARBEITUNGSVORRICHTUNG, INFORMATIONSVERARBEITUNGSVERFAHREN UND PROGRAMM
DISPOSITIF DE TRAITEMENT D'INFORMATIONS, PROCÉDÉ DE TRAITEMENT D'INFORMATIONS ET PROGRAMME

(30) Priority: 25.10.2016 JP 2016208781
(43) Date of publication of application: 04.09.2019
(73) Proprietor: Sony Group Corporation, Tokyo 108-0075 (JP)
(72) Inventor: WAKITA, Yoshihiro, Tokyo 108-0075 (JP); MIYASHITA, Ken, Tokyo 108-0075 (JP); OKUBO, Atsushi, Tokyo 108-0075 (JP); KAWAMOTO, Yohei, Tokyo 108-0075 (JP)
(74) Representative: MFG Patentanwälte Meyer-Wildhagen Meggle-Freund Gerhard PartG mbB
(86) International application number: PCT/JP2017/027352
(87) International publication number: WO 2018/078983

(56) References cited:
- WO-A1-2016/054079
- JP-A- 2014 041 426
- JP-A- 2016 028 747
- JP-A- 2016 176 678
- JP-A- H06 245 914
- JP-A- H1 043 148
- US-A- 5 991 697
- US-A1- 2010 082 302
- US-A1- 2014 316 292
- US-A1- 2016 302 735

## Description

### Technical Field

The present disclosure relates to an information processing apparatus, an information processing method, and a program.

### Background Art

Techniques for measuring information regarding blood flow such as a pulse and a blood flow velocity are frequently used in the field of medicine and the like. As an example of an apparatus for measuring a pulse and a blood flow velocity, a blood flowmeter can be mentioned. A blood flowmeter can be installed on a measured person without giving discomfort, pain, or the like to the measured person and easily measure the pulse and the blood flow velocity. For example, an example of a blood flowmeter is disclosed in Patent Literature 1.

The US patent application publication US 2010/0082302 A1 discloses methods and apparatuses for signal processing, for example, for sensor signal processing, e.g., in Body Area Networks for healthcare and fitness applications. More specifically this document discloses reconstructing a PPG signal using a compressed sensing approach which allows to heavily under-sample the original PPG data while still being able to estimate the original PPG data with a high fidelity. This may help for reducing power consumption of a pulse oximeter sensor.

### Citation List

### Patent Literature

Patent Literature 1: JP 2013-146371A

### Disclosure of Invention

### Technical Problem

In the above-described blood flowmeter, a signal on which a noise component is superimposed is sometimes detected in a case in which a sampling frequency is lowered to suppress power consumption. For example, in a case in which a sampling frequency does not satisfy a predetermined condition for a frequency of a signal obtained from blood flow, noise sometimes occurs due to a folding phenomenon. Then, in a case in which a process is performed on a detection signal on which such a noise component is superimposed, it is difficult to obtain accurate blood flow information such as a pulse due to the noise component.

Accordingly, the present disclosure is devised in view of the foregoing circumstances and proposes an information processing apparatus, an information processing method, and a program capable of obtaining accurate blood flow information while suppressing power consumption.

### Solution to Problem

According to a first aspect, the present invention provides an information processing apparatus in accordance with independent claim 1. According to a second aspect, the present invention provides a computer-implemented method in accordance with independent claim 5. According to a third aspect, the present invention provides a computer program in accordance with claim 6. Further aspects of the present invention are set forth in the dependent claims, the drawings and the following description.

According to the present disclosure, there is provided an information processing apparatus including: an estimation unit configured to estimate another kind of blood flow information associated with one kind of blood flow information from the one kind of blood flow information obtained through blood flow measurement on the basis of relation information indicating a relation between the two different kinds of blood flow information.

In addition, according to the present disclosure, there is provided an information processing method including: estimating another kind of blood flow information associated with one kind of blood flow information from the one kind of blood flow information obtained through blood flow measurement on the basis of relation information indicating a relation between the two different kinds of blood flow information.

Furthermore, according to the present disclosure, there is provided a program causing a computer to realize: a function of estimating another kind of blood flow information associated with one kind of blood flow information from the one kind of blood flow information obtained through blood flow measurement on the basis of relation information indicating a relation between the two different kinds of blood flow information.

### Advantageous Effects of Invention

According to the present disclosure, as described above, it is possible to obtain accurate blood flow information while suppressing power consumption.

Note that the effects described above are not necessarily limitative. With or in the place of the above effects, there may be achieved any one of the effects described in this specification or other effects that may be grasped from this specification.

### Brief Description of Drawings

[FIG. 1] FIG. 1 is a block diagram illustrating a functional configuration of an information processing system 1 according to an embodiment of the present disclosure.
[FIG. 2] FIG. 2 is an explanatory diagram illustrating an example of an operation pattern of a radiation unit 100 and a detection unit 102 according to the embodiment of the present disclosure.
[FIG. 3] FIG. 3 is a diagram illustrating an example of a form of a measurement module 10 according to an embodiment of the present disclosure.
[FIG. 4] FIG. 4 is an explanatory diagram for describing a form when the measurement module 10 illustrated in FIG. 3 is installed.
[FIG. 5] FIG. 5 is an explanatory diagram illustrating a blood flow measurement method applied to the embodiment of the present disclosure.
[FIG. 6] FIG. 6 is an explanatory diagram illustrating a first processing method applied to the embodiment of the present disclosure.
[FIG. 7] FIG. 7 is an explanatory diagram illustrating a second processing method applied to the embodiment of the present disclosure.
[FIG. 8] FIG. 8 is an explanatory diagram illustrating a power spectrum 606 on which folding noise components 602 and 604 are superimposed.
[FIG. 9] FIG. 9 is a block diagram illustrating a functional configuration of a processor 300 of an information processing apparatus 30 according to a first embodiment of the present disclosure.
[FIG. 10] FIG. 10 is an explanatory diagram illustrating an information processing method according to the first embodiment of the present disclosure.
[FIG. 11] FIG. 11 is a diagram illustrating of a flowchart of a first operation in the information processing method according to the first embodiment of the present disclosure.
[FIG. 12] FIG. 12 is a diagram illustrating of a flowchart of a second operation in the information processing method according to the first embodiment of the present disclosure.
[FIG. 13] FIG. 13 is a block diagram illustrating a functional configuration of a processor 300a of the information processing apparatus 30 according to a modification example of the first embodiment of the present disclosure.
[FIG. 14] FIG. 14 is a block diagram illustrating a functional configuration of a processor 300b of the information processing apparatus 30 according to a second embodiment of the present disclosure.
[FIG. 15] FIG. 15 is an explanatory diagram illustrating an information processing method according to a second embodiment of the present disclosure.
[FIG. 16] FIG. 16 is an explanatory diagram illustrating an information processing method according to a modification example of the second embodiment of the present disclosure.
[FIG. 17] FIG. 17 is a block diagram illustrating a functional configuration of a processor 300c of the information processing apparatus 30 according to a third embodiment of the present disclosure.
[FIG. 18] FIG. 18 is an explanatory diagram illustrating an information processing method according to the third embodiment of the present disclosure.
[FIG. 19] FIG. 19 is a block diagram illustrating a functional configuration of a processor 300d of the information processing apparatus 30 according to a fourth embodiment of the present disclosure.
[FIG. 20] FIG. 20 is an explanatory diagram illustrating an information processing method according to the fourth embodiment of the present disclosure.
[FIG. 21] FIG. 21 is a diagram illustrating of a flowchart of the information processing method according to the fourth embodiment of the present disclosure.
[FIG. 22] FIG. 22 is a block diagram illustrating a configuration of the information processing apparatus 30 according to an embodiment of the present disclosure.

### Mode(s) for Carrying Out the Invention

Hereinafter, (a) preferred embodiment(s) of the present disclosure will be described in detail with reference to the appended drawings. Note that, in this specification and the appended drawings, structural elements that have substantially the same function and structure are denoted with the same reference numerals, and repeated explanation of these structural elements is omitted.

The description will be made in the following order.
1. Configuration of information processing system 1 according to embodiment of present disclosure
   1.1 Configuration of measurement module 10
   1.2 Configuration of information processing apparatus 30
2. Blood flow measurement method and processing method according to embodiment of present disclosure
   2.1 Blood flow information measurement method
   2.2 Processing methods
      2.2.1 First processing method
      2.2.2 Second processing method
3. Background of embodiment of present disclosure
4. First embodiment
   4.1 Configuration of processor 300 according to first embodiment
   4.2 Information processing method according to first embodiment
      4.2.1 First operation
      4.2.2 Second operation
   4.3 Modification example of first embodiment
5. Second embodiment
   5.1 Configuration of processor 300b according to second embodiment
   5.2 Information processing method according to second embodiment
   5.3 Modification example of second embodiment
6. Third embodiment
   6.1 Configuration of processor 300c according to third embodiment
   6.2 Information processing method according to third embodiment
7. Fourth embodiment
   7.1 Configuration of processor 300d according to fourth embodiment
   7.2 Information processing method according to fourth embodiment
8. Hardware configuration
9. Supplement

### <<1. Configuration of information processing system 1 according to embodiment of present disclosure>>

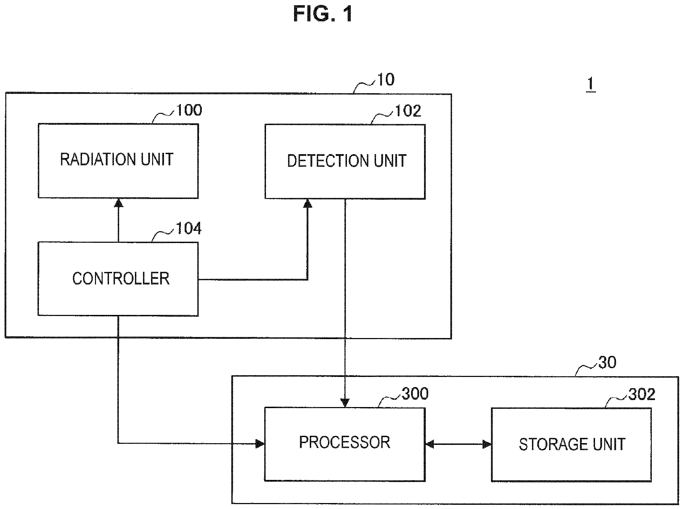

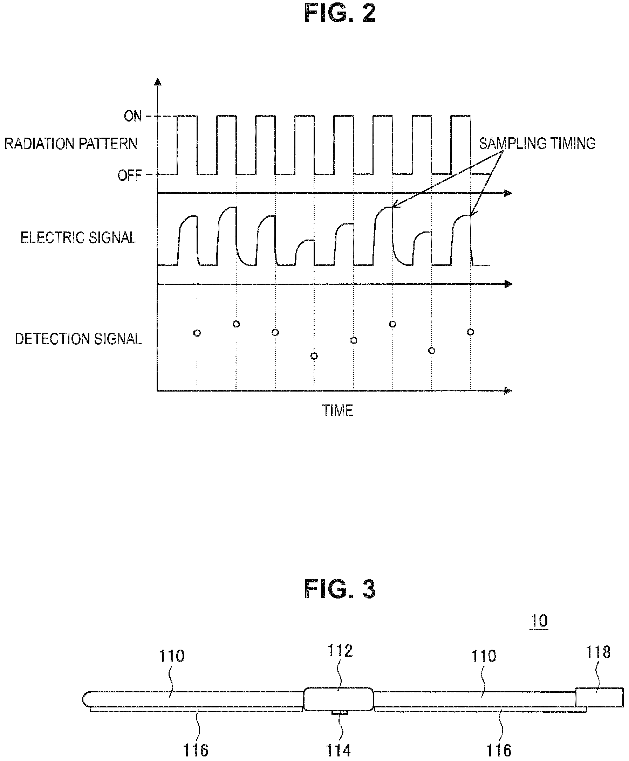

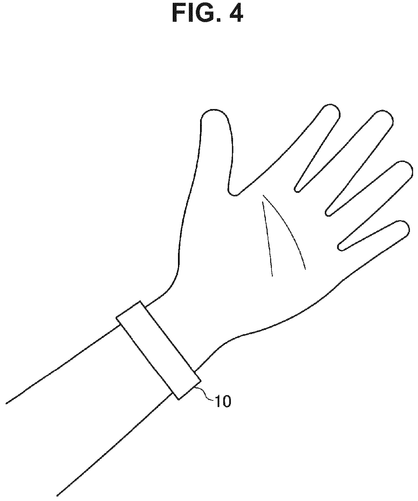

First, an information processing system 1 according to an embodiment of the present disclosure will be described with reference to FIGS. 1 to 4. FIG. 1 is a block diagram illustrating a functional configuration of an information processing system 1 according to an embodiment of the present disclosure. FIG. 2 is an explanatory diagram illustrating an example of an operation pattern of a radiation unit 100 and a detection unit 102 according to the embodiment of the present disclosure. FIG. 3 is a diagram illustrating an example of a form of a measurement module (measurement unit) 10 according to an embodiment of the present disclosure. Further, FIG. 4 is an explanatory diagram for describing a form when the measurement module 10 illustrated in FIG. 3 is installed.

According to the embodiment of the present disclosure, blood flow measurement is performed to acquire blood flow information regarding blood flow of a measured person. Specifically, the blood flow information refers to information regarding blood flow, such as a pulse rate, an average blood flow velocity, a blood flow amount, a velocity distribution of particles in a blood vessel, or the like. Further, in the following description, the blood flow information also includes blood flow signal information of a power spectrum or the like used to calculate the above-described information such as a pulse obtained by processing a detection signal obtained through blood flow measurement. In addition, the pulse rate refers to the number of pulsations of an artery per unit time appearing on a body surface or the like due to occurrence of a change in pressure on an inner wall of the artery when muscles of a heart contract at a regular rhythm (pulsation, and further the number of pulsations in the heart per unit time is referred to as a pulse rate) to send blood to the whole body through the artery. Further, the blood flow velocity refers to a velocity of blood (blood component) flowing in one blood vessel or a plurality of blood vessels in a measurement region which is a measured person and a blood flow amount refers to a blood amount passing per unit time through one blood vessel or a plurality of blood vessels in the measurement region. The velocity distribution of particles in blood vessels refers to a velocity distribution of density of particles staying or flowing in blood vessels such as red blood cells in one blood vessel or a plurality of blood vessels in a measurement region. In addition, blood can be considered to be a mixture of substances with a plurality of flow velocities and a feature of blood flow can be indicated by a motion of blood cells which are particles in a blood vessel, that is, a flow velocity of the blood cells. The flow velocity of the blood cells can be used as a main index indicating the feature of the blood flow. In the present disclosure, an average moving velocity of blood cells (particles) in blood is called an average blood flow velocity.

In addition, in the embodiment of the present disclosure, to acquire the above-described blood flow information, light is radiated to a part (measurement region) of a measured person such as a hand, an arm, a neck, or a leg and light scattered in substances moving in blood vessels or a stationary biological tissue of the measured person is detected. Here, in the embodiment, blood flow information is acquired by processing detected light (in particular, a detection signal). Note that, in the embodiment to be described below, a case in which a pulse rate is acquired as a result of blood flow measurement will be described as an example. However, in the embodiment of the present disclosure, other blood flow information may be acquired as a result of the blood flow measurement without being limited to a case in which the pulse rate is acquired as a result of the blood flow measurement.

The information processing system 1 according to the embodiment mainly includes the measurement module 10 and an information processing apparatus 30, as illustrated in FIG. 1. Further, the information processing system 1 according to the embodiment may include an information presentation apparatus that notices a measurement result or the like to a user (the user may be a measured person who is a target of blood flow measurement or a person or the like using the information processing system 1 according to the embodiment other than the measured person) and is not illustrated in FIG. 1. Hereinafter, configurations of the measurement module 10 and the information processing apparatus 30 included in the information processing system 1 will be described sequentially.

### <1.1 Configuration of measurement module 10>

The measurement module 10 according to the embodiment is a module that is mounted on a part of the body such as skin of the measured person to perform blood flow measurement on the measured person. As illustrated in FIG. 1, the measurement module 10 mainly includes a radiation unit 100, the detection unit 102, and a controller 104. Hereinafter, each functional unit included in the measurement module 10 will be described.

### (Radiation unit 100)

The radiation unit 100 radiates radiation light with a predetermined wavelength to a measurement region (a part of the body) of the measured person. The wavelength of the radiation light radiated by the radiation unit 100 can be appropriately selected and, for example, light with a wavelength around 850 nm is radiated. As the radiation unit 100, a small laser or the like can be used to radiate coherent light. Then, the controller 104 to be described below can control a timing, a radiation time, a radiation interval, a strength, and the like at which the radiation light of the radiation unit 100 is radiated.

### (Detection unit 102)

The detection unit 102 detects light scattered from the measurement region of the measured person. The detection unit 102 includes, for example, a photodiode (photo detector: PD), converts the strength of the received light into an electric signal, and outputs the electric signal to the information processing apparatus 30 to be described below. Note that a charge coupled device (CCD) type sensor, a complementary metal oxide semiconductor (CMOS) type sensor, or the like can be used as the detection unit 102. In addition, the single photodiode, sensor, or the like or the plurality of photodiodes, sensors, or the like described above are provided in the measurement module 10. Further, a timing or the like at which the detection unit 102 outputs (reads) a detection signal is controlled by the controller 104 to be described below.

### (Controller 104)

The controller 104 controls general measurement in the measurement module 10 by controlling a radiation pattern (a radiation timing, a radiation time, and a radiation interval) of the radiation unit 100, controlling a reading (sampling) timing of the detection unit 102, or the like on the basis of a predetermined synchronization signal or the like. For example, the controller 104 controls a radiation frequency of the radiation unit 100 or a sampling frequency of the detection unit 102 synchronized with the radiation frequency in accordance with an operation of the information processing system 1. In addition, the controller 104 may further include a storage unit (not illustrated) and the storage unit may store various programs, parameters, or the like for controlling the radiation unit 100 or the like. Further, the controller 104 may contain a clock mechanism (not illustrated) that ascertains an accurate time to output a detection signal to the information processing apparatus 30 in association with a time. Specifically, the controller 104 is realized by, for example, a central processing unit (CPU), a read-only memory (ROM), a random access memory (RAM), and the like. Note that some or all of the functions performed by the controller 104 may be performed by the information processing apparatus 30 to be described below.

Here, the details of control on the radiation unit 100 and the detection unit 102 by the above-described controller 104 will be described with reference to FIG. 2. FIG. 2 is an explanatory diagram illustrating an example of an operation pattern of the radiation unit 100 and the detection unit 102 according to the embodiment of the present disclosure. In particular, the upper stage of FIG. 2 schematically illustrates a radiation pattern of the radiation unit 100, the middle stage of FIG. 2 illustrates an electric signal detected by the detection unit 102, and the lower stage of FIG. 2 illustrates a detection signal obtained through sampling of the detection unit 102. Note that in the following description, sampling refers to digitizing and reading (outputting) an electric signal generated in the detection unit 102 due to detection of light using, for example, an analog-digital converter or the like.

As illustrated in the upper stage of FIG. 2, the radiation pattern of the radiation unit 100 is a square wave. In particular, the radiation unit 100 radiates light during a period of a flat portion (ON) of the upper side (a radiation period). On the other hand, the radiation unit 100 pauses the radiation during a period of a flat portion (OFF) of the lower side (a pause period). In addition, as illustrated in the middle stage of FIG. 2, a sampling timing of the detection unit 102 is synchronized with the radiation period of the radiation unit 100. In particular, during the radiation period, the detection unit 102 changes the electric signal in accordance with an amount of light received in the detection unit 102 and samples a change in the electric signal generated in the detection unit 102 at a timing at which the radiation unit 100 pauses the radiation. That is, in the right end portion of a peak of a reading pattern in the middle stage of FIG. 2, a change in the electric signal generated in accordance with the amount of light received in the detection unit 102 can be read and the detection signal illustrated in the lower stage of FIG. 2 can be obtained. Note that, in the following description, the number of samplings per unit time is called a sampling frequency. Note that, in the following description, an example in which the sampling is performed at an equal time interval is used to describe the present disclosure in brief, but the sampling can also be performed at an unequal time interval by using a principle of compressed sensing or the like. The sampling frequency of this case can be considered to be an average value of the number of samplings in a certain time section.

Note that radiation and sampling patterns in a radiation and measurement method according to the embodiment of the present disclosure are not limited to the patterns illustrated in FIG. 2. For example, in the radiation pattern of the radiation unit 100, a radiation section in which the radiation unit 100 repeats the radiation a predetermined number of times regularly at a first interval may be repeated at a second interval longer than the first interval. Note that, even in this case, the sampling timing of the detection unit 102 is synchronized with the radiation of the radiation unit 100. That is, in the embodiment, various radiation and sampling patterns can be selected in accordance with desired blood flow information, measurement accuracy, and the like.

Further, although not illustrated in FIG. 1, the measurement module 10 has a power source supplying power to the radiation unit 100 or the like. Further, the measurement module 10 may include a communication unit (not illustrated) or the like communicating with the information processing apparatus 30 or the like to be described below in addition to the radiation unit 100, the detection unit 102, and the controller 104 described above. In addition, the measurement module 10 may include a sensor (not illustrated) such as a pressure sensor detecting that the measurement module 10 is mounted on a part of the body of the measured person.

In addition, the measurement module 10 can have, for example, the form of a wearable apparatus mounted on the body of the measured person for use. For example, the measurement module 10 may be a device that has the shape of a wrist-watch, a ring, a wristband, an anklet, a necklace, an earphone, or the like and can be mounted on a part of the measured person such as a wrist, an arm, a neck, a leg, or an ear. In addition, the measurement module 10 may be a device that has a pad shape such as a sticking plaster and can be pasted to a part of the measured person such as a hand, an arm, a neck, or a leg. Further, the measurement module 10 may have an implant shape embedded in a part of the body of the measured person.

Hereinafter, an example of a specific form of the measurement module 10 according to the embodiment will be described with reference to FIGS. 3 and 4. For example, as illustrated in FIG. 3, the measurement module 10 can have the form of a belt shape. As illustrated in FIG. 3, the measurement module 10 includes a band unit 110, a control unit 112, and a measurement unit 114 in a belt shape. The control unit 112 is a portion in which the above-described controller 104 is provided. Note that in a case in which the measurement module 10 and the information processing apparatus 30 to be described below are an integrated apparatus, each functional unit to be described below in the information processing apparatus 30 may be provided in the control unit 112. In addition, the measurement unit 114 is a portion in which the radiation unit 100 and the detection unit 102 described above are provided and comes into contact with or faces the body of the measured person when the measurement module 10 is mounted on a part of the body.

The band unit 110 is, for example, a component that fixes the measurement module 10 to be wrapped around a wrist of the measured person and is formed of a material such as a soft silicone gel to form a ring shape that conforms to the shape of the wrist. That is, since the band unit 110 can be formed in a ring shape that conforms to the shape of the wrist, as illustrated in FIG. 4, the measurement module 10 is wrapped to be fixed around the wrist of the measured person. In addition, when the measurement module 10 is moved during blood flow measurement, accurate measurement may not be performed. Therefore, the measurement module 10 is preferably fixed on a measurement region of the measured person. Accordingly, an adhesive layer 116 which can be adhered to skin of the measured person may be provided in a portion of the band unit 110 coming into contact with the skin of the measured person. Further, it is preferable to freely adjust a circumferential length of a ring when the measurement module 10 is formed in the ring shape to correspond to thicknesses of various wrists. Accordingly, the fixing unit 118 is provided at an end of the band unit 110 and the fixing unit 118 can be superimposed on any portion on the band unit 110 to be fixed at various positions on the band unit 110. In this way, the measurement module 10 can be mounted in accordance with the thickness of the wrist of the measured person to be fixed.

### <1.2 Configuration of information processing apparatus 30>

Referring back to FIG. 1, a configuration of the information processing apparatus 30 of the information processing system 1 according to the embodiment will be described. The information processing apparatus 30 is an apparatus that acquires blood flow information such as a pulse using a detection signal obtained by the measurement module 10. As illustrated in FIG. 1, the information processing apparatus 30 mainly includes a processor 300 and a storage unit 302. Hereinafter, each functional unit included in the information processing apparatus 30 will be described.

### (Processor 300)

The processor 300 acquires blood flow information by processing the detection signal obtained by the measurement module 10. The acquired blood flow information can be output to the storage unit 302 to be described below or another apparatus. Note that the details of the processor 300 will be described later.

### (Storage unit 302)

The storage unit 302 stores a program or various kinds of data to be used in a process in the above-described processor 300 and stores the blood flow information or the like acquired by the processor 300. In addition, in addition to the data or the like, the storage unit 302 may appropriately store various parameters, ongoing progress of a process, and the like necessarily stored when any process is performed. Then, the processor 300 or the like can freely access the storage unit 302 to write or read data.

Note that the information processing apparatus 30 may also include a communication unit (not illustrated) communicating with the measurement module 10 or the like in addition to the above-described processor 300 and storage unit 302. Further, the information processing apparatus 30 may include an input unit (not illustrated) or the like receiving a manipulation from a user using the information processing system 1 according to the embodiment.

In addition, the information processing apparatus 30 may be an apparatus integrated with the above-described measurement module 10 or may be an apparatus separate from the above-described measurement module 10. In the latter case, for example, the information processing apparatus 30 may be an information processing apparatus such as a smartphone, a tablet, or a personal computer (PC) or may be an information processing apparatus connected to another apparatus (for example, a medical apparatus or the like). Further, the information processing apparatus 30 may be an information processing apparatus such as a server installed in a location away from the measured person or the like.

### <<2. Blood flow measurement method and processing method according to embodiment of present disclosure>>

As described above, in the embodiment of the present disclosure, the blood flow measurement is performed to acquire the blood flow information regarding blood flow of the measured person. As described above, in the embodiment, to acquire the above-described blood flow information, light is radiated to a part of a measured person such as a hand, an arm, a neck, or a leg, light scattered in substances moving in blood vessels or a stationary biological tissue of the measured person is detected, and the detected light (in particular, a detection signal) is processed. Hereinafter, a blood flow measurement method, a processing method, and the like according to the embodiment of the present disclosure will be described in detail.

### <2.1 Blood flow information measurement method>

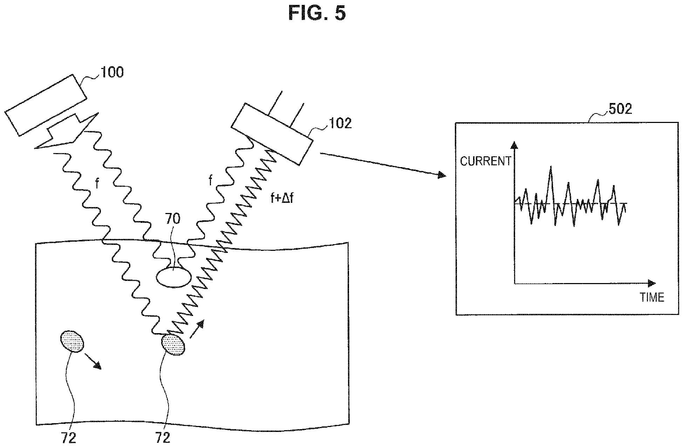

An analysis technology for a velocity distribution using a laser Doppler blood flow measurement technology or a dynamic light scattering (DLS) technique can be exemplified as an example of the blood flow measurement method according to the embodiment of the present disclosure. First, an interference phenomenon of coherent light for blood flow common to both the technologies will be described with reference to FIG. 5. FIG. 5 is an explanatory diagram illustrating a blood flow measurement method applied to the embodiment of the present disclosure. In particular, FIG. 5 schematically illustrates an interference phenomenon of coherent light by blood flow. Reference numeral 502 in FIG. 5 denotes an example of a waveform of a detection signal obtained through the measurement.

The blood flow information measurement method according to the embodiment of the present disclosure is a method of using the phenomenon that light scattered by the scattering substances (mainly, red blood cells) moving in blood vessels of the measured person produces interference light by the Doppler effect and location movement of scattering substances when light from the radiation unit 100 is radiated to a measurement region (a part of the body) of the measured person. The interference light is received by the detection unit 102 such as a photodiode and blood flow information is calculated from a distribution of a Doppler shift frequency in the received interference light.

In particular, as illustrated in FIG. 5, in a case in which light with a frequency f radiated to a measurement region of the measured person by the radiation unit 100 is scattered by a situated stationary tissue 70 such as skin or a subcutaneous tissue of the measured person, the scattered light maintains the frequency f. On the other hand, in a case in which the light with the frequency f radiated to the measurement region of the measured person is scattered by scattering substances (for example, red blood cells can be exemplified and the red blood cells are a substance with a diameter of 8 to 10 µm) 72 moving in blood vessels of the measured person (for example, moving particles causing Doppler shift in the scattered light), the frequency of the scattered light is shifted by the Doppler effect and location movements of the scattering substances, and thus the scattered light has a frequency f+Δf. Then, the scattered light with the frequency f scattered by the stationary tissue 70 interferes with the scattered light with the frequency f+Δf scattered by the moving scattering substances 72, and thus the detection unit 102 can detect the interference light with optical beats. Note that the shift frequency Δf is considerably smaller than the frequency f of the radiated light.

Then, by processing the interference light (detection signal) detected by the detection unit 102 and denoted by reference numeral 502 in FIG. 5, it is possible to obtain blood flow information. Note that, since the detection signal 502 is a signal in which optical beats with a plurality of different frequencies by the scattered light from the particles performing a plurality of different movements in the blood vessels are superimposed, as illustrated in FIG. 5, the detection signal 502 is seen as an irregular signal such as white noise. However, the detection signal 502 is a signal in which the interference beats with the plurality of frequencies are superimposed, as described above. Therefore, by performing a frequency analyzing process to be described below on the detection signal, it is possible to acquire velocity distribution information of particle movements causing Doppler shift. In the present disclosure, since an observation target is blood flow, a velocity distribution of particles such as the red blood cells in blood vessels can be ascertained. In addition, since blood flow information in a biological tissue within a range in which the radiated light arrives can be acquired, blood flow information in a region including blood vessels in a deep part located to a certain depth from skin of the body of the measured person as well as blood vessels of the surface of the skin of the measured person can be acquired.

### <2.2 Processing methods>

Further, in the embodiment of the present disclosure, blood flow information is acquired by processing a detection signal detected by the detection unit 102, as described above. In the embodiment of the present disclosure, for example, any of two methods to be described below can be used as a method of processing a detection signal to acquire blood flow information. In particular, as the processing method according to the embodiment, a first processing method in which a frequency analyzing process (Fourier transform) used generally for laser Doppler velocity detection is first performed and a second processing method of calculating an autocorrelation function used in a DLS technique can be exemplified. Hereinafter, the details of the first and second processing methods will be described sequentially.

### <2.2.1 First processing method>

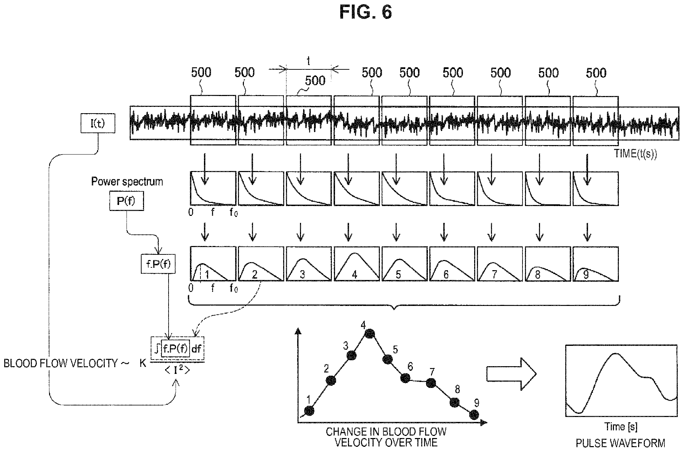

First, the first processing method will be described with reference to FIG. 6. FIG. 6 is an explanatory diagram illustrating the first processing method applied to the embodiment. In the first processing method, as illustrated in FIG. 6, blood flow information is acquired by first performing, for example, a frequency analyzing process such as a fast Fourier transform (FFT) on the detection signal (I(t) of FIG. 6) obtained by the detection unit 102 for each interval of a plurality of ranges (windows 500 of FIG. 6).

Specifically, in the first processing method, an FFT is performed on the detection signal at each interval of a predetermined time range to acquire a plurality of power spectra (P(f) of FIG. 6) which are a function of a frequency. Further, by taking a product of a beat frequency having a proportional relation with a velocity for each frequency in each of the acquired power spectra (fP(f) of FIG. 6) and performing integration in the entire power spectra and normalization, it is possible to obtain an average blood flow velocity. Then, by acquiring a plurality of blood flow velocities on the basis of the plurality of power spectra obtained from the plurality of windows with different time ranges, it is possible to acquire a waveform indicating a change in the average blood flow velocity over time. Further, by performing an interpolation process or the like on the acquired waveform, a pulse waveform indicating a change in the blood flow velocity by pulse can be acquired and a pulse rate or the like can be calculated from the pulse waveform. Note that, in FIG. 6, the plurality of superimposed windows 500 deciding the ranges for generating the power spectra are not expressed. However, in an actual process, the windows 500 can be caused to be mutually superimposed. Thus, by processing the windows 500 caused to be superimposed, it is possible to more densely generate the plurality of power spectrum lines that are formed chronologically.

### <2.2.2 Second processing method>

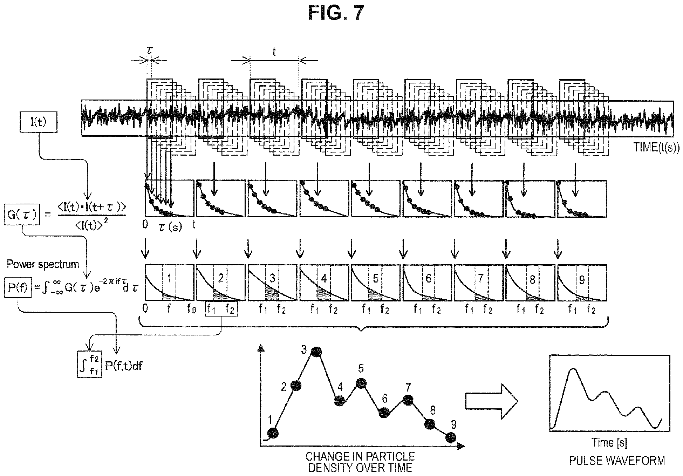

Next, the second processing method will be described with reference to FIG. 7. FIG. 7 is an explanatory diagram illustrating the second processing method applied to the embodiment. In the second processing method, as illustrated in FIG. 7, blood flow information is acquired by first calculating an autocorrelation function from the detection signal (I(t) of FIG. 7) and processing the calculated autocorrelation function (G(τ) of FIG. 7).

In particular, according to the Wiener-Khichin theorem, the power spectra of I(t) are acquired by performing Fourier transform on the autocorrelation function after the autocorrelation function is obtained. Note that according to the second processing method in which the autocorrelation function is used, the accurate power spectra can be obtained even in a case in which a detected detection signal is a signal that does not have periodicity as in the present disclosure. Here, by processing the acquired power spectra, it is possible to acquire desired blood flow information.

More specifically, in the second processing method, calculation of the autocorrelation function is performed on the detection signal for each predetermined time range to acquire the plurality of autocorrelation functions. Further, in the second processing method, according to the Wiener-Khichin theorem, the FFT is performed on each of the calculated autocorrelation function to acquire a plurality of power spectra (P(f) of FIG. 7) which are a function of a frequency. The power spectra are proportional to existence density of particles moving at velocities corresponding to the frequencies of the power spectra. Therefore, by performing an integration process on the acquired power spectra in a predetermined frequency range, it is possible to obtain relative densities of the particles in the blood vessels within a predetermined velocity range. Further, by acquiring the relative densities of the plurality of particles from the plurality of power spectra with different time ranges, it is possible to acquire a waveform indicating a change in the relative densities of the particles over time. Then, by performing an interpolation process or the like on the obtained waveform, it is possible to acquire a pulse waveform indicating a change in the relative densities of the particles by pulse and calculate a pulse rate or the like from the pulse waveform. Note that in the above-described first and second processing methods, different methods are used in the way of obtaining the power spectra and the way of obtaining the blood flow information subsequently, but any combination can be used in the present disclosure. That is, in the present disclosure, after the power spectra are obtained in accordance with the method described in the first processing method, the relative densities of the particles within the predetermined velocity range indicated by the second processing method can also be obtained. In addition, in the present disclosure, after the power spectra are obtained in accordance with the method described in the second processing method, the average blood flow velocity indicated in the first processing method can also be obtained.

### <<3. Background of embodiment of present disclosure>>

Incidentally, since the measurement module 10 according to the embodiment of the present disclosure is a wearable apparatus mounted on the measured person, the measurement module 10 is preferably compact. Therefore, it is preferable to reduce a power source volume of the measurement module 10. Accordingly, in order to reduce the power source volume, power consumption in the measurement module 10 is required to be suppressed as small as possible. Further, considering that the measurement module 10 is mounted on the measured person for a long time to be able to perform blood flow measurement for a long time as much as possible, power consumption in the measurement module 10 is required to be suppressed as small as possible.

**In** the measurement module 10, the power consumption is considerably consumed by the radiation unit 100. Accordingly, in order to suppress the power consumption, it is considered that the number of radiations of light is reduced and furthermore the number of samplings (sampling frequency) synchronized with the radiation is reduced. **In** other words, by lowering the sampling frequency, it is possible to reduce a radiation period (the number of radiations or a time) of the radiation unit 100 synchronized with the sampling, and thus it is possible to suppress the power consumption in the radiation unit 100.

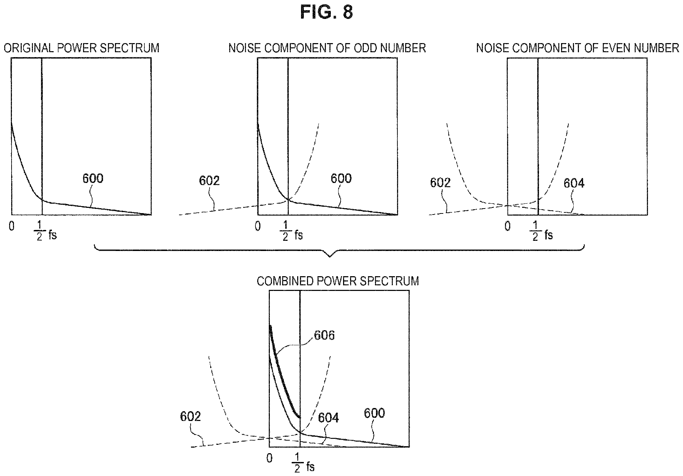

However, a signal with a frequency equal to or greater than 1/2 of a sampling frequency and included in an original signal is mixed as a folding signal with a discretely sampled signal according to the Nyquist theorem. When the sampling frequency is lowered, the folding signal increases and shortly reaches the magnitude which may not be negligible. Thus, as a noise signal, the folding signal has an adverse influence on extraction of information (blood flow information in the present disclosure). Specifically, in the method described in the embodiment of the present disclosure, a folding noise component is superimposed on the power spectrum, the noise component has an adverse influence on a process at the rear stage, and thus it is difficult to obtain accurate flood flow information. First, a power spectrum 606 on which folding noise components 602 and 604 are superimposed will be described with reference to FIG. 8. FIG. 8 is an explanatory diagram illustrating the power spectrum 606 on which the folding noise components 602 and 604 are superimposed.

First, an original power spectrum 600 of an original signal is illustrated in FIG. 8. **In** addition, when fs is a sampling frequency, the noise component 602 with a waveform in which the waveform of the original signal is folded at a position of 1/2fs according to the Nyquist theorem occurs, as indicated as a noise component by an odd number of folding in FIG. 8. Further, a noise component in which the folding noise component is further folded at a position corresponding to a multiple of a Nyquist frequency also occurs. For example, the noise component 604 that has a waveform in which the noise component 602 is folded at a position of a frequency corresponding to a zero multiple of the Nyquist frequency (that is, the frequency is zero) occurs to appear as a noise component by folding of an even number in FIG. 8. Then, the noise components 602 and 604 are superimposed on the original power spectrum 600 and a power spectrum 606 on which the noise components are superimposed is detected to appear as a combined power spectrum in FIG. 8. Further, in a case in which the above-described process is performed on the power spectrum 606 on which such folding noise components 602 and 604 are superimposed, it is difficult to acquire accurate blood flow information since the noise components 602 and 604 are superimposed. Note that the folding of two times has been described herein. However, since the folding continues up to the infinity actually, high-frequency components included in an original signal are all convoluted in a signal with 1/2 fs or less.

Note that the folding noise component 604 occurring at a position of a frequency of an even multiple of the Nyquist frequency has a shape in which the power spectrum 600 on which no noise component is superimposed is translated along the frequency axis (the X axis). Accordingly, it is easy to suppose an influence of the noise component 604 from the detection signal (the power spectrum 606 on which the noise component is superimposed) and the influence on calculation of the blood flow information can be said to be small. **In** contrast, the folding noise component 602 occurring at a position of a frequency of an odd multiple of the Nyquest frequency has a shape in which power spectrum 600 on which no noise component is superimposed is inverted using the corresponding position of the Nyquist frequency as a mirror plane. Accordingly, it is difficult to suppose an influence of the noise component 602 from the detection signal (the power spectrum 606 on which the noise component is superimposed) and the influence on calculation of the blood flow information can be said to be large. **In** addition, for the blood flow information corresponding to a frequency range (or a velocity range) considerably influenced by the folding noise components, of course, it is difficult to acquire accurate information due to the folding noise components.

That is, in a case in which the sampling frequency is lowered to suppress power consumption in the measurement module 10, the folding noise components are superimposed on the detection signal, and thus it is difficult to obtain the accurate blood flow information from the detection signal due to the folding noise components.

Accordingly, the present inventors have created embodiments of the present disclosure in view of the foregoing circumstances. According to the embodiments of the present disclosure, it is possible to obtain accurate blood flow information even in a case in which a detection signal on which folding noise components are superimposed since a sampling frequency is lowered to suppress power consumption of the measurement module 10 is acquired. In particular, according to the embodiments of the present disclosure, blood flow information such as a power spectrum which is not influenced from the folding noise components is estimated from the detection signal on which the folding noise components are superimposed. Then, according to the embodiments, more desired blood flow information is acquired by processing the estimated blood flow information such as a power spectrum. Accordingly, according to the embodiment of the present disclosure, it is possible to obtain the accurate blood flow information by using the estimated blood flow information such as the power spectrum which is not influenced from the folding noise components even in a case in which the sampling frequency is lowered. Hereinafter, detailed configurations and operations of the embodiments of the present disclosure will be described sequentially in detail.

### <<4. First embodiment>>

In a first embodiment, a power spectrum on which a folding noise component is greatly superimposed since a sampling frequency is lowered is detected, but a power spectrum in a satisfactory state in which the folding noise component is negligible is estimated from the power spectrum. Accordingly, in the embodiment, to perform the above-described estimation, the information processing apparatus 30 performs machine learning of a relation between a power spectrum on which no folding noise component is superimposed and a power spectrum on which a folding noise component corresponding to the power spectrum is superimposed. Then, on the basis of relation information obtained through the machine learning, the information processing apparatus 30 estimates a power spectrum which corresponds to the power spectrum and which is in a satisfactory state in which the folding noise component is negligible, from the power spectrum on which a separately acquired folding noise component is superimposed. Further, in the embodiment, a pulse rate which is desired blood flow information is acquired using the estimated power spectrum.

In particular, in the embodiment, to perform the machine learning of the relation information, blood flow measurement is performed at a high sampling frequency (hereinafter referred to as a first sampling frequency) when a power spectrum in a satisfactory state in which a folding noise component is negligible (hereinafter referred to as a first power spectrum) is acquired. By setting the first sampling frequency to be twice or more a frequency at which a noise component can be sufficiently attenuated to the extent that an adverse influence of a folding phenomenon is negligible, it is possible to avoid the adverse influence of the noise component occurring in the folding phenomenon by the Nyquist theorem. Hereinafter, a frequency at which the noise component is sufficiently attenuated to the extent that the adverse influence of the folding phenomenon is negligible is referred to as a maximum frequency. It is possible to acquire a first detection signal (a first blood flow signal) in a state in which the adverse influence of the folding noise component is negligible through the blood flow measurement at the first sampling frequency which is twice or more the maximum frequency and a first power spectrum by further processing the first detection signal. The first sampling frequency is a frequency equal to or greater than 16 kHz and is, for example, a frequency of about 100 kHz. Note that power consumption in the measurement module 10 increases in the blood flow measurement at the first sampling frequency.

In the embodiment, with regard to the first detection signal in the state in which the adverse influence of the folding noise component is negligible, a second detection signal (second blood flow signal) corresponding to a low sampling frequency (hereinafter referred to as a second sampling frequency) is acquired by performing a process of decimating some of the signals included in the first detection signal in accordance with a predetermined rule (a decimation process). That is, the decimation process is performed to acquire a signal equal to a detection signal acquired in a case in which the process is performed at the second sampling frequency at the time of the blood flow measurement in which the above-described first detection signal is acquired. The second sampling frequency is a frequency selected to suppress the power consumption of the measurement module 10, is particularly less than twice the maximum frequency, and is a frequency less than the above-described first sampling frequency. Accordingly, since the second sampling frequency is equal to or less than twice the maximum frequency, a folding noise component with a level which has an adverse influence on calculation of the blood flow information is superimposed on the second detection signal. Then, a power spectrum on which a folding noise component is superimposed (hereinafter referred to as a second power spectrum) is acquired by processing the second detection signal on which the folding noise component is superimposed. For example, as the second sampling frequency, a frequency equal to or less than 1/2 of the above-described first sampling frequency can be selected and, more specifically, a frequency with about several kHz to several 10 kHz can be selected.

Then, in the embodiment, a relation between the first power spectrum and the second power spectrum obtained as described above is obtained by machine learning. Specifically, in the machine learning, relation information indicating the relation between the first power spectrum and the second power spectrum is acquired by comparing waveforms in accordance with a predetermined rule or deriving a mathematical correspondent relation. Since a velocity distribution of particles during blood flow has a monotonous phenomenon property smaller as the particles of which velocities are generally faster and a distribution pattern in which restriction is imposed on various conditions caused from the shape of the blood vessels or viscosity of blood is shown, there are many limitations on the distribution shape of the power spectrum. Accordingly, it is possible to acquire the relation information from the above-described machine learning.

Further, in the embodiment, a power spectrum on which a folding noise component is superimposed (hereinafter referred to as a third power spectrum) is acquired from a third detection signal on which the folding noise component detected in another blood flow measurement is superimposed. Note that the foregoing another blood flow measurement is performed at the low second sampling frequency to suppress power consumption in the measurement module 10. Then, on the basis of the relation information obtained through the above-described machine learning, a power spectrum which corresponds to the third power spectrum and is in a satisfactory state in which the folding noise component is negligible (hereinafter referred to as a fourth power spectrum) is estimated from the acquired third power spectrum. As described above, the velocity distribution of the particles during the blood flow has a particular nature and there is limitation on the distribution shape of the power spectrum. Accordingly, on the basis of the relation information obtained through the above-described machine learning, the fourth power spectrum which corresponds to the third power spectrum and is in a satisfactory state in which the folding noise component is negligible can be estimated from the third power spectrum on which the folding noise component is superimposed. Note that the fourth power spectrum corresponds to a power spectrum obtained in a case of the actual measurement at the first sampling frequency in the foregoing other blood flow measurement.

Note that in the following description, an operation when the first and second detection signals are acquired through the blood flow measurement and then the relation information is acquired through the machine learning is referred to as a first operation. Further, in the following description, an operation when the third detection signal is acquired through another blood flow measurement, the fourth power spectrum is estimated, and then blood flow information (for example, a pulse rate) is calculated from the estimated fourth power spectrum is referred to as a second operation. In the embodiment, in the first operation, power consumption in the measurement module 10 is high since the measurement is performed at the high first sampling frequency. In the embodiment, however, by performing the first operation only at a predetermined timing rather than in every blood flow measurement, it is possible to suppress the power consumption in the measurement module 10. Then, in the embodiment, the second operation is an operation at the time of normal blood flow measurement. By performing the blood flow measurement at the low second sampling frequency, it is possible to suppress the power consumption in the measurement module 10. That is, by performing the second operation in every blood flow measurement, it is possible to suppress the power consumption. Hereinafter, detailed configurations and operations according to the embodiment will be described sequentially in detail.

Note that since the configurations of the information processing system 1, the measurement module 10, and the information processing apparatus 30 according to the embodiment have been described above, the description thereof will be omitted herein.

### <4.1 Configuration of processor 300 according to first embodiment>

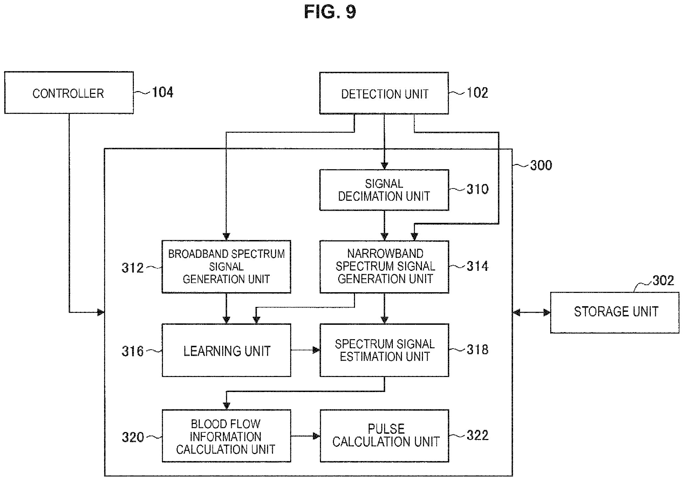

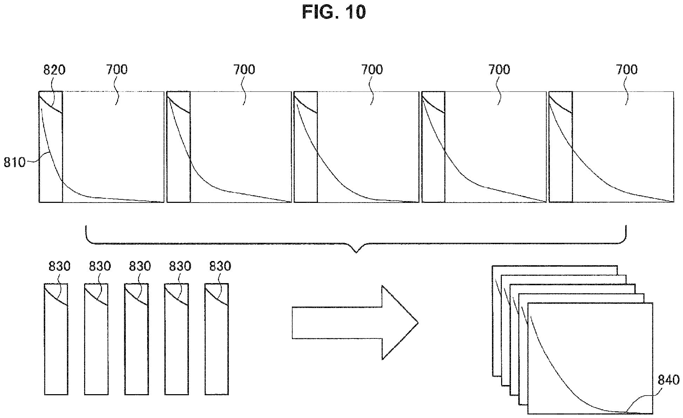

Hereinafter, the details of the processor 300 according to the embodiment will be described with reference to FIGS. 9 and 10. FIG. 9 is a block diagram illustrating a functional configuration of the processor 300 of the information processing apparatus 30 according to the embodiment. FIG. 10 is an explanatory diagram illustrating an information processing method according to the embodiment. As described above, the processor 300 acquires desired blood flow information by processing a detection signal obtained by the measurement module 10. In particular, as illustrated in FIG. 9, the processor 300 mainly includes an inter-signal decimation unit 310, a broadband spectrum signal generation unit 312, a narrowband spectrum signal generation unit 314, a learning unit 316, a spectrum signal estimation unit 318, a blood flow information calculation unit 320, and a pulse calculation unit 322. Hereinafter, each functional unit included in the processor 300 will be described.

### (Inter-signal decimation unit 310)

The inter-signal decimation unit 310 performs a process of decimating some of signals included in the first detection signal in accordance with a predetermined rule on the first detection signal acquired at the first sampling frequency in the first operation to acquire the second detection signal corresponding to the second sampling frequency. The acquired second detection signal is output to the narrowband spectrum signal generation unit 314 to be described below.

### (Broadband spectrum signal generation unit 312)

The broadband spectrum signal generation unit 312 performs a process on the first detection signal detected by the detection unit 102 of the measurement module 10 to generate a first power spectrum 810 (see FIG. 10). In particular, the broadband spectrum signal generation unit 312 performs the FFT on the first detection signal to generate the first power spectrum 810 (the first processing method). Alternatively, the broadband spectrum signal generation unit 312 calculates an autocorrelation function from the first detection signal and performs the FFT on the calculated autocorrelation function to generate the first power spectrum 810 (the second processing method).

More specifically, the broadband spectrum signal generation unit 312 performs a process on the first detection signal obtained at the first sampling frequency in the first operation to generate the first power spectrum 810. The generated first power spectrum 810 is output to the learning unit 316 to be described below so that the first power spectrum 810 is supplied for machine learning. Note that in the first power spectrum 810, an adverse influence of a folding noise component on the calculation of the blood flow information is small to the extent that the adverse influence is negligible since the first sampling frequency is sufficiently high.

### (Narrowband spectrum signal generation unit 314)

The narrowband spectrum signal generation unit 314 performs a process on the second detection signal processed by the inter-signal decimation unit 310 to generate a second power spectrum 820 (see FIG. 10). In particular, the narrowband spectrum signal generation unit 314 performs the FFT on the second detection signal processed by the inter-signal decimation unit 310 to generate the second power spectrum 820 as in the above-described broadband spectrum signal generation unit 312 (the first processing method). Alternatively, the narrowband spectrum signal generation unit 314 calculates an autocorrelation function from the second detection signal and performs the FFT on the calculated autocorrelation function to generate the second power spectrum (the second processing method). On the other hand, the narrowband spectrum signal generation unit 314 performs a process on the third detection signal detected at the second sampling frequency in the second operation to generate a third power spectrum 830 (see FIG. 10). Note that a folding noise component is greatly superimposed on the third power spectrum 830 to the extent that the folding noise component has an adverse influence on the calculation of the flood flow information since the second sampling frequency is low. The generated third power spectrum 830 is output to the spectrum signal estimation unit 318 to be described below in order to estimate a fourth power spectrum 840 (see FIG. 10) which is in a state in which the folding noise component is small to the extent that the adverse influence is negligible.

More specifically, the narrowband spectrum signal generation unit 314 performs a process on the second detection signal corresponding to the second sampling frequency and processed by the inter-signal decimation unit 310 in the first operation to generate the second power spectrum 820. The generated second power spectrum 820 is output to the learning unit 316 to be described below so that the second power spectrum 820 is supplied for the machine learning. Note that a folding noise component is greatly superimposed on the second power spectrum 820 to the extent that the folding noise component has an adverse influence on the calculation of the blood flow information since the second sampling frequency is low. On the other hand, in the second operation, the narrowband spectrum signal generation unit 314 performs a process on the third detection signal obtained at the second sampling frequency to generate the third power spectrum 830. The generated third power spectrum 830 is supplied to the spectrum signal estimation unit 318 so that the fourth power spectrum 840 is estimated.

### (Learning unit 316)

The learning unit 316 performs the machine learning using the first power spectrum 810 output from the broadband spectrum signal generation unit 312 and the second power spectrum 820 output from the narrowband spectrum signal generation unit 314 in the first operation. Then, information (relation information) obtained through the machine learning in the learning unit 316 is stored in the storage unit 302 so that the information is used in the spectrum signal estimation unit 318 to be described below.

In particular, the first power spectrum 810 output from the broadband spectrum signal generation unit 312 is the power spectrum generated from the first detection signal corresponding to the first sampling frequency, as described above. Accordingly, in the first power spectrum 810, a folding noise component is small to the extent that the adverse influence of the folding noise component on the calculation of the blood flow information is negligible since the first sampling frequency is sufficiently high. As a result, by processing the first power spectrum 810 in the state in which the folding noise component is small to the extent that the adverse influence is negligible, it is possible to acquire the accurate blood flow information. In contrast, the second power spectrum 820 output from the narrowband spectrum signal generation unit 314 is a power spectrum generated from a signal corresponding to the second sampling frequency, as described above. Accordingly, a folding noise component is superimposed on the second power spectrum 820 to the extent that the folding noise component has an adverse influence on the calculation of the blood flow information since the second sampling frequency is low. As a result, as described, it is difficult to directly process the second power spectrum 820 on which the folding noise component is greatly superimposed and acquire the accurate blood flow information.

Accordingly, the learning unit 316 performs the machine learning of the relation between the first power spectrum 810 and the second power spectrum 820. Specifically, the learning unit 316 performs leaning in a supervised leaner such as a support vector regression or a deep neural network using the first power spectrum 810 and the second power spectrum 820 as a supervised signal and an input signal, respectively, in accordance with a predetermined rule. Further, the learning unit 316 acquires relation information indicating a relation between the first power spectrum 810 and the second power spectrum 820 by the above-described learning. Since the velocity distribution of the particles during the blood flow has a particular nature and there is limitation on the distribution shape of the power spectrum, it is possible to find specific relation information from the above-described machine learning. For example, as illustrated in the upper stage of FIG. 10, the learning unit 316 acquires the relation information by the machine learning using one power spectrum pair 700 formed by the first power spectrum 810 and the second power spectrum 820 or a plurality of power spectrum pairs 700.

### (Spectrum signal estimation unit 318)

The spectrum signal estimation unit 318 estimates the fourth power spectrum 840 from the third power spectrum 830 acquired by the blood flow measurement on the basis of the relation information regarding the blood flow information obtained by the learning unit 316 in the second operation. In particular, the third power spectrum includes a folding noise component and the estimated fourth power spectrum 840 corresponds to the third power spectrum. Then, the fourth power spectrum 840 estimated by the spectrum signal estimation unit 318 is output to the blood flow information calculation unit 320 to be described below. In particular, the spectrum signal estimation unit 318 acquires the third power spectrum 830 corresponding to the second sampling frequency and output from the narrowband spectrum signal generation unit 314 as an input signal in the second operation. Then, on the basis of the relation information obtained through the machine learning of the learning unit 316, the spectrum signal estimation unit 318 estimates the fourth power spectrum (the power spectrum corresponding to the first sampling frequency) 840 in the state in which the adverse influence of the folding noise component is negligible, from the third power spectrum 830. For example, the spectrum signal estimation unit 318 estimates each fourth power spectrum 840 illustrated in the lower stage of FIG. 10 on the basis of relation learning (see the upper stage of FIG. 10) from each third power spectrum 830 illustrated in the lower stage of FIG. 10.

### (Blood flow information calculation unit 320)

The blood flow information calculation unit 320 calculates blood flow information (a blood flow velocity, a particle density in blood vessels within a predetermined velocity range, or the like) using the fourth power spectrum 840 estimated by the spectrum signal estimation unit 318 in the second operation. Then, the blood flow information calculated by the blood flow information calculation unit 320 is output to the pulse calculation unit 322 to be described below. In particular, the blood flow information calculation unit 320 can obtain the blood flow velocity or the like by integrating values obtained by taking products of frequencies having proportional relations with particle velocities in the entire power spectrum in the fourth power spectrum 840 and subsequently performing normalization. Then, the blood flow information calculation unit 320 acquires a waveform indicating a change in the blood flow velocity over time by acquiring the plurality of blood flow velocities from the plurality of fourth power spectra 840 (the first processing method). Alternatively, the blood flow information calculation unit 320 can obtain a relative density of the particles in a predetermined velocity range in the blood vessels by performing the integration on the fourth power spectrum 840 in a predetermined frequency range. Further, the blood flow information calculation unit 320 acquires a waveform indicating a relative density of the particles over time by acquiring the relative density of the plurality of particles from the plurality of fourth power spectra 840 calculated from a plurality of different time ranges (the second processing method).

### (Pulse calculation unit 322)

The pulse calculation unit 322 calculates a pulse waveform by performing an interpolation process or the like on the waveform obtained from the blood flow information calculation unit 320 and calculates a pulse rate from the pulse waveform in the above-described second operation. The pulse rate or the like obtained by the pulse calculation unit 322 may be output to the storage unit 302 or may be output to the above-described information posting apparatus (not illustrated).

### <4.2 Information processing method according to first embodiment>

Next, an information processing method according to the first embodiment of the present disclosure will be described. The information processing method according to the embodiment can be broadly divided into the first operation and the second operation described above. In the first operation, the first power spectrum 810 corresponding to the high first sampling frequency and the second power spectrum 820 corresponding to the low second sampling frequency are acquired and machine learning of this relation is performed. In addition, in the second operation, the third power spectrum 830 corresponding to the second sampling frequency is acquired. Then, the fourth power spectrum 840 which is in a state the adverse influence of the folding noise component is negligible, that is, which corresponds to the first sampling frequency, is estimated from the third power spectrum 830. Further, in the second operation, desired blood flow information is acquired from the estimated fourth power spectrum 840. Accordingly, hereinafter, the information processing method according to the embodiment is divided into the first operation and the second operation for the description.

### <4.2.1 First operation>

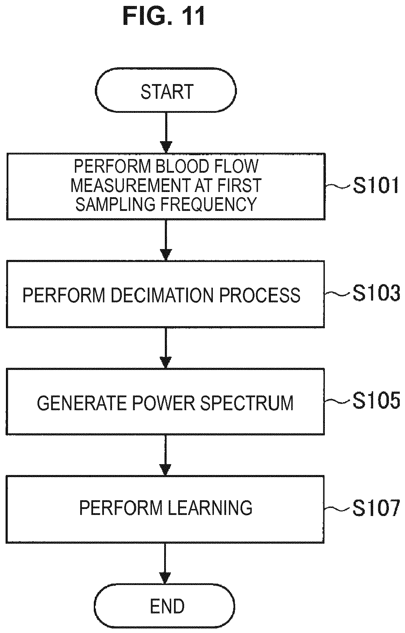

First, a first operation of the information processing method according to the embodiment will be described with reference to FIG. 11. FIG. 11 is a diagram illustrating of a flowchart of the first operation in the information processing method according to the embodiment. As illustrated in FIG. 11, the first operation in the information processing method according to the embodiment includes step S101 to step S107. Hereinafter, each step of the first operation will be described.

First, before the first operation starts, for example, as illustrated in FIG. 4, the above-described measurement module 10 is mounted on a wrist or the like of the measured person. Note that, for example, the first operation may be performed when the measurement module 10 is mounted on a part of the body of the measured person or may be performed when the measured person performs first measurement. In addition, the first operation may be performed when the blood flow measurement starts or may be performed for each predetermined period (10 minutes or 20 minutes) in the blood flow measurement. Alternatively, the first operation may be performed when a manipulation by a user using the information processing system 1 is received.

### (Step S101)

When it is detected that the measurement module 10 is mounted on a part of the body of the measured person, or the like, the measurement module 10 starts the blood flow measurement. Then, the information processing apparatus 30 acquires a first detection signal corresponding to the first sampling frequency. Note that the blood flow measurement may be performed a plurality of times by repeating step S101.

### (Step S103)

The information processing apparatus 30 performs the decimation process on the first detection signal corresponding to the first sampling frequency and detected in step S101 on the basis of a predetermined rule to acquire a second detection signal corresponding to the second sampling frequency.

### (Step S105)

The information processing apparatus 30 performs a process on the first detection signal corresponding to the first sampling frequency and detected in step S101 to generate the first power spectrum 810. Further, the information processing apparatus 30 performs a process on the second detection signal corresponding to the second sampling frequency and acquired in step S103 to generate the second power spectrum 820. Note that in a case in which the blood flow measurement is performed a plurality of times by repeating the above-described step S101, step S103 and step S105 are repeated the plurality of times.

In this way, by performing step S101 to step S105 once or a plurality of times, it is possible to obtain one power spectrum pair 700 formed by the first power spectrum 810 and the second power spectrum 820, as illustrated in the upper stage of FIG. 10 or the plurality of power spectrum pairs 700.

### (Step S107)

The information processing apparatus 30 performs machine learning of the relation between the first power spectrum 810 and the second power spectrum 820 using the power spectrum pair 700 generated in step S105. Then, the information processing apparatus 30 stores the relation information regarding the first power spectrum 810 and the second power spectrum 820 obtained through the machine learning as information which is used for the second operation. Note that the number of power spectrum pairs 700 used for the learning of step S107 may be 1 or plural and the power spectrum pair 700 can be selected in accordance with the accuracy of the desired blood flow information or the like.

Note that the above-described first operation may not be performed in the information processing system 1 and the relation information acquired in another information processing system 1 may be stored in advance in the storage unit 302 when the information processing apparatus 30 is manufactured or shipped, or the like. In this case, the second operation, that is, the normal blood flow measurement, can be immediately performed without performing the first operation. In addition, by repeating the above-described first operation using data measured in measured people with a plurality of different features, it is possible to obtain performance for stably estimating power spectra for diverse measured people. Further, in the embodiment, by performing the first operation at a specific timing during the blood flow measurement, it is also possible to perform the learning in real time. In this case, since the learning in accordance with the blood flow features of the measured people can be performed, the accuracy of the estimation can be further improved than in a case in which the estimation is performed on the basis of only learning results of other measured people. Furthermore, by performing the first operation when the measurement module 10 is mounted, the learning can be performed in accordance with a mounting state (the measurement module 10 is mounted tightly or mounted loosely on a part of the body of the measured person, or the like) or a mounted part of the measurement module 10. Therefore, it is possible to further improve accuracy of the estimation.

### <4.2.2 Second operation>

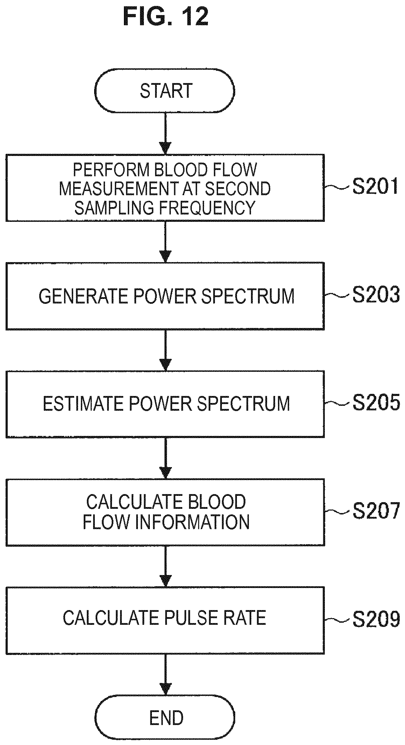

Next, the second operation of the information processing method according to the embodiment will be described with reference to FIG. 12. FIG. 12 is a diagram illustrating of a flowchart of the second operation in the information processing method according to the embodiment. As illustrated in FIG. 12, the second operation in the information processing method according to the embodiment includes step S201 to step S209. Hereinafter, each step of the second operation will be described.

First, before the second operation starts, for example, as illustrated in FIG. 4, the above-described measurement module 10 is mounted on a wrist or the like of the measured person.

### (Step S201)

When it is detected that the measurement module 10 is mounted on a part of the body of the measured person, or the like, the measurement module 10 starts the blood flow measurement. Then, the information processing apparatus 30 acquires the third detection signal at the second sampling frequency. Note that the blood flow measurement can be continuously performed by continuously repeating step S201.

### (Step S203)

The information processing apparatus 30 performs a process on the third detection signal corresponding to the second sampling frequency and detected in step S201 to generate the third power spectrum 830. Note that in a case in which the blood flow measurement is performed a plurality of times by repeating the above-described step S201, step S203 is repeated the plurality of times. **In** this way, by performing step S201 to step S203 the plurality of times, it is possible to obtain the plurality of third power spectra 830, as illustrated in the lower stage of FIG. 10. Thus, it is possible to continuously acquire the third power spectra 830 chronologically.

### (Step S205)

The information processing apparatus 30 estimates the fourth power spectrum 840 from the third power spectrum 830 on which a folding noise component is superimposed and which is obtained in step S203 on the basis of the relation information obtained in step S107 of the first operation. The estimated fourth power spectrum 840, the adverse influence of the folding noise component is small to the extent that the folding noise component is negligible.

### (Step S207)

The information processing apparatus 30 acquires the desired blood flow information using the fourth power spectrum 840 estimated in step S205.

### (Step S209)

The information processing apparatus 30 calculates a pulse rate from the blood flow information obtained in step S207. Note that the pulse rate obtained in step S209 may be output to the storage unit 302 or may be output to the above-described information posting apparatus (not illustrated).

As described above, in the embodiment, the third power spectrum 830 on which the folding noise component is superimposed since the sampling frequency is lowered is detected. Accordingly, in the embodiment, the machine learning is performed on the relation between the first power spectrum 810 which is in the state in which the adverse influence of the folding noise component is negligible and the second power spectrum 820 which corresponds to the first power spectrum 810 and on which the folding noise component is greatly superimposed. Then, in the embodiment, the relation information indicating the relation is acquired. Further, on the basis of the relation information, the fourth power spectrum 840 which is in the state in which the adverse influence of the folding noise component is negligible is estimated from the foregoing third power spectrum 830 and the desired blood flow information (a pulse rate or the like) is acquired using the estimated fourth power spectrum 840. Accordingly, in the embodiment, it is possible to obtain the highly accurate blood flow information from the third power spectrum 830 on which the folding noise component is superimposed since the sampling frequency is lowered, while suppressing the power consumption of the measurement module 10 by lowering the sampling frequency.

### <4.3 Modification example of first embodiment>

In the above-described first embodiment, in step S103 of the first operation, the decimation process has been performed on the first detection signal to obtain the second detection signal corresponding to the second sampling frequency. However, in the embodiment, the present disclosure is not limited to this form. For example, two detection units 102 may be caused to operate simultaneously to acquire the first detection signal corresponding to the first sampling frequency and the second detection signal corresponding to the second sampling frequency. In this case, the processor 300 can reduce a processing amount of the processor 300 since the signal decimation process is not performed. Hereinafter, this embodiment will be described as a modification example of the above-described first embodiment.

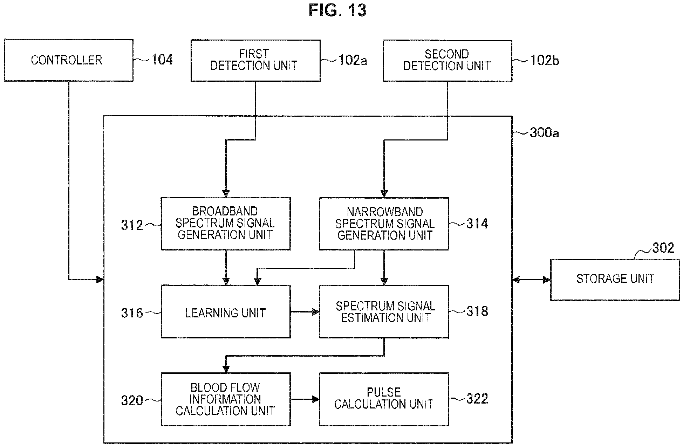

In the modification example, compared to the configuration according to the first embodiment, two detection units 102 are provided and further the inter-signal decimation unit 310 is omitted in a processor 300a. In particular, as illustrated in FIG. 13 which is a block diagram illustrating a functional configuration of the processor 300a according to the modification example, a first detection unit 102a and a second detection unit 102b are provided and the inter-signal decimation unit 310 is not provided in the processor 300a in the modification example. The first detection unit 102a performs the blood flow measurement at the first sampling frequency in the first operation and outputs the first detection signal to the broadband spectrum signal generation unit 312. Further, the first detection unit 102a performs the blood flow measurement at the second sampling frequency in the second operation and outputs the third detection signal to the narrowband spectrum signal generation unit 314. In addition, the second detection unit 102b performs the blood flow measurement at the second sampling frequency in the first operation and directly outputs the second detection signal to the narrowband spectrum signal generation unit 314. That is, in the modification example, in step S101 in FIG. 11 illustrating a flowchart of the first operation of the information processing method according to the above-described first embodiment, the blood flow measurement at the first and second sampling frequencies is performed. Further, in the modification example, the process subsequently proceeds to step S105 of generating the power spectrum without performing step S103 in FIG. 11.

For example, equipment in which the two detection units 102a and 102b according to the modification example illustrated in FIG. 13 are provided is assumed to be used, for example, when learning data for machine learning gathers in advance. Further, as products to be mass-produced, equipment in which one detection unit 102b performing the blood flow measurement at the second sampling frequency is provided is assumed. In this way, in the mass-produced products, one detection unit 102 that operates at the low second sampling frequency may be provided. Therefore, it is possible to decrease manufacturing cost of the products.

### <<5. Second embodiment>>

In the above-described first embodiment, the power spectrum (the fourth power spectrum 830) has been estimated. In an embodiment, however, blood flow information such as a blood flow velocity may be estimated without being limited to the estimation of the power spectra. Hereinafter, the embodiment will be described as a second embodiment. In the following description, an average blood flow velocity will be estimated. In the embodiment, by directly estimating an average blood flow velocity, it is possible to reduce a processing amount in the second operation. Note that since the configurations of the information processing system 1, the measurement module 10, and the information processing apparatus 30 according to the embodiment have been described above, the description thereof will be omitted.

### <5.1 Configuration of processor 300b according to second embodiment>

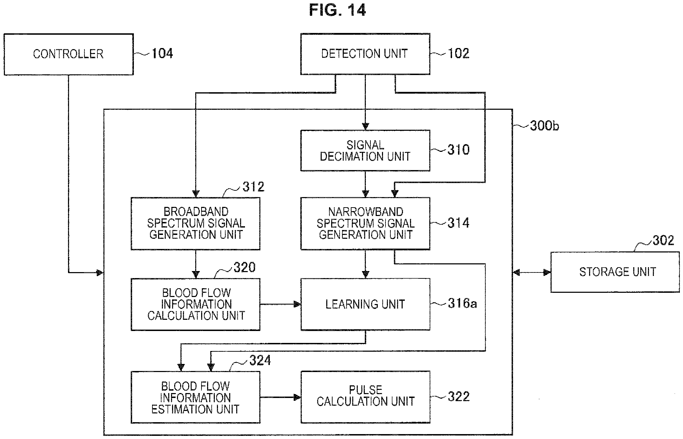

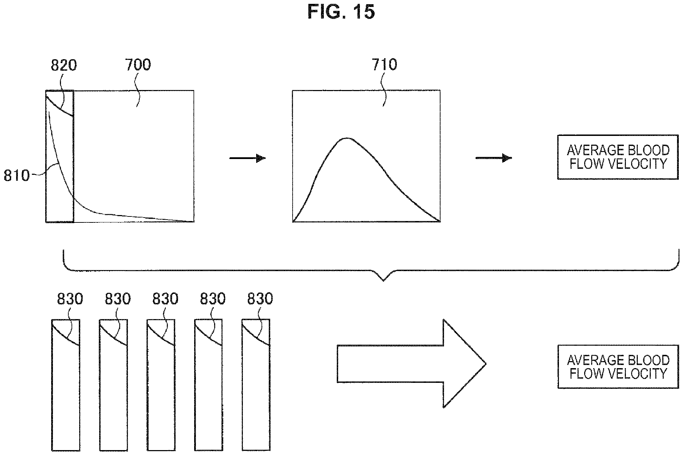

In the embodiment, each functional unit included in the processor 300b is different from that of the processor 300 according to the first embodiment. Hereinafter, a configuration of the processor 300b according to the embodiment will be described with reference to FIGS. 14 and 15. FIG. 14 is a block diagram illustrating a functional configuration of the processor 300b according to the embodiment. FIG. 15 is an explanatory diagram illustrating an information processing method according to a second embodiment.

As illustrated in FIG. 14, the processor 300b mainly includes the inter-signal decimation unit 310, the broadband spectrum signal generation unit 312, the narrowband spectrum signal generation unit 314, a learning unit 316a, the blood flow information calculation unit 320, the pulse calculation unit 322, and a blood flow information estimation unit 324. That is, in the embodiment, the learning unit 316a and the blood flow information estimation unit 324 are different from those of the first embodiment. Accordingly, the description of the common functional units to those of the first embodiment will be omitted and only an association relation between the functional units, the learning unit 316a, and the blood flow information estimation unit 324 will be described.

### (Association relation between functional units in processor 300b according to second embodiment)

In the second embodiment, a calculation result of the broadband spectrum signal generation unit 312 is output to the blood flow information calculation unit 320. In addition, a calculation result of the narrowband spectrum signal generation unit 314 is output to the learning unit 316a and the blood flow information estimation unit 324. A calculation result of the blood flow information calculation unit 320 is output to the learning unit 316a. A calculation result of the learning unit 316a is used when the blood flow information estimation unit 324 performs estimation via the storage unit 302. Further, an estimation result of the blood flow information estimation unit 324 is output to the pulse calculation unit 322.

### (Learning unit 316a)

The learning unit 316a performs machine learning using an average flood flow velocity (a first average blood flow velocity) output from the blood flow information calculation unit 320 and the second power spectrum 820 output from the narrowband spectrum signal generation unit 314 in the above-described first operation. Then, relation information between the average blood flow velocity and the second power spectrum 820 obtained through the machine learning in the learning unit 316a is stored in the storage unit 302 to be used in the blood flow information estimation unit 324 to be described below. Note that the average blood flow velocity output to the learning unit 316a is obtained by processing the first detection signal in which a folding noise component is small to the extent that an adverse influence of the folding noise component is negligible. More specifically, as illustrated in the upper stage of FIG. 15, the learning unit 316a acquires an average blood flow velocity from a blood flow velocity distribution 710 acquired from the first power spectrum 810. Further, the learning unit 316a acquires the second power spectrum 820 forming the power spectrum pair 700 along with the first power spectrum 810. Then, the learning unit 316a learns a relation between the average blood flow velocity and the second power spectrum 820. In other words, the learning unit 316a learns a relation between the second power spectrum 820 on which the folding noise component is superimposed and the average blood flow velocity on which there is no influence of the folding noise component. Note that one second power spectrum 820 with which a correspondent relation is learned by the learning unit 316a may be used or the plurality of second power spectra 820 with different time ranges may be used. In an example of the blood flow information estimation unit 324 to be described below, a relation between one average blood flow velocity and the second power spectra 820 with five different time ranges is learned (see FIG. 15).

### (Blood flow information estimation unit 324)

The blood flow information estimation unit 324 estimates an average blood flow velocity from the third power spectrum 830 on which a folding noise component is superimposed on the basis of the relation information obtained by the learning unit 316a in the second operation. Then, the average blood flow velocity estimated by the blood flow information estimation unit 324 is output to the above-described pulse calculation unit 322a. For example, the blood flow information estimation unit 324 estimates an average blood flow velocity illustrated in the lower stage of FIG. 15 on the basis of relation learning (see the upper stage of FIG. 15) from each third power spectrum 830 illustrated in the lower stage of FIG. 15. Here, for example, the blood flow information estimation unit 324 estimates the corresponding average blood flow velocity from the five power spectra 830 with the different time ranges.

### <5.2 Information processing method according to second embodiment>

Next, an information processing method according to the embodiment will be described. The information processing method according to the embodiment can be divided into a first operation and a second operation as in the first embodiment. First, the first operation of the information processing method according to the embodiment will be described with reference to FIG. 11 which is a flowchart of the first operation according to the first embodiment. The first operation according to the embodiment is different from that of the first embodiment in that the average blood flow velocity is calculated in addition to generation of the power spectrum in step S105 illustrated in FIG. 11. Further, the first operation according to the embodiment is different from that of the first embodiment in that in step S107 illustrated in FIG. 11, the relation between the first power spectrum 810 and the second power spectrum 820 is not learned, and the relation between the average blood flow velocity and the second power spectrum 820 is learned.

Next, the second operation of the information processing method according to the embodiment will be described with reference to FIG. 12 which is the flowchart of the second operation according to the first embodiment. The second operation according to the embodiment is different from that of the first embodiment in that the average blood flow velocity is estimated instead of estimating the third power spectrum 830 in step S205 illustrated in FIG. 12.

As described above, in the embodiment, the third power spectrum 830 on which the folding noise component is superimposed since the sampling frequency is lowered is detected as in the first embodiment. Accordingly, in the embodiment, the average blood flow velocity obtained from the first power spectrum 810 in which the folding noise component is small to the extent that the adverse influence of the folding noise component is negligible is acquired. Further, the relation information is acquired by performing the machine learning of the relation between the average blood flow velocity and the power spectrum 820 on which the folding noise component is superimposed and which corresponds to the first power spectrum 810. Then, in the embodiment, on the basis of the relation information, the average blood flow velocity on which there is no influence of the folding noise component is estimated from the foregoing third power spectrum 830 and desired blood flow information (pulse rate or the like) is acquired using the estimated average blood flow velocity. Accordingly, in the embodiment, it is possible to obtain the accurate blood flow information from the power spectrum 830 on which the folding noise component is superimposed since the sampling frequency is lowered, while suppressing the power consumption of the measurement module 10 by lowering the sampling frequency. Furthermore, in the embodiment, it is possible to reduce a processing amount in the second operation because of the direct estimation of the average blood flow velocity.

### <5.3 Modification example of second embodiment>

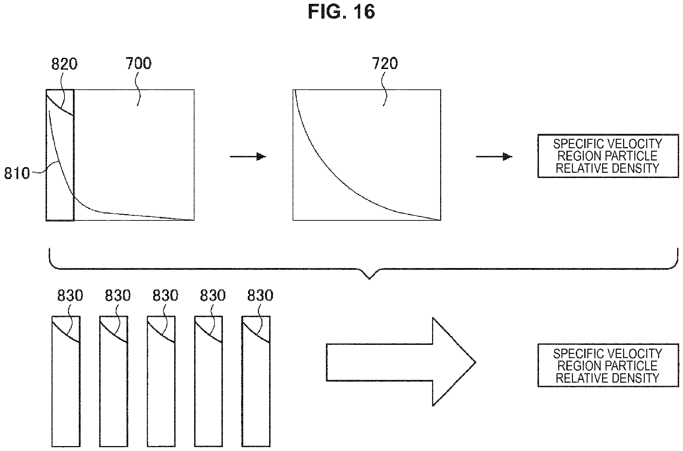

In the above-described second embodiment, the average blood flow velocity has been estimated. However, the embodiment is not limited to this form. For example, a relative density of particles in a predetermined velocity range may be estimated. Hereinafter, a modification example of the embodiment in which a relative density of the particles is estimated will be described with reference to FIG. 16 which is an explanatory diagram illustrating an information processing method according to the modification example of the embodiment.

In the first operation according to the modification example, as illustrated in the upper stage of FIG. 16, the first detection signal corresponding to the first sampling frequency is acquired, a process is performed on the first detection signal, and a distribution 720 of a particle density in blood vessels is acquired. Further, in the modification example, a relative density (a first specific velocity range particle relative density) of the particles in the blood vessels in a predetermined velocity range is calculated from the particle density distribution 720. In addition, in the modification example, the second detection signal corresponding to the second sampling frequency is acquired and the second power spectrum 820 is generated as in the first and second embodiments. Then, in the modification example, machine learning of a relation between the second power spectrum 820 and the first specific velocity range particle relative density is performed.

Further, in the second operation according to the modification example, the third detection signal corresponding to the second sampling frequency is acquired and the acquired third detection signal is processed to acquire the third power spectrum 830 as in the first and second embodiments (see the lower stage of FIG. 16). Then, in the modification example, a specific velocity range particle relative density (a second specific velocity range particle relative density) is estimated from the third power spectrum 830 on the basis of the relation information (see the upper stage of FIG. 16) obtained through the machine learning (see the lower stage of FIG. 16).

### <<6. Third embodiment>>

In the above-described first and second embodiments, the power spectrum, the average blood flow velocity, and the like have been estimated. In an embodiment, however, a pulse rate may be directly estimated without being limited to the estimation of the power spectrum or the like. Hereinafter, the embodiment will be described as a third embodiment. In the embodiment, by directly estimating a pulse rate, it is possible to reduce a processing amount in the second operation. Note that since the configurations of the information processing system 1, the measurement module 10, and the information processing apparatus 30 according to the embodiment have been described above, the description thereof will be omitted.

### <6.1 Configuration of processor 300c according to third embodiment>

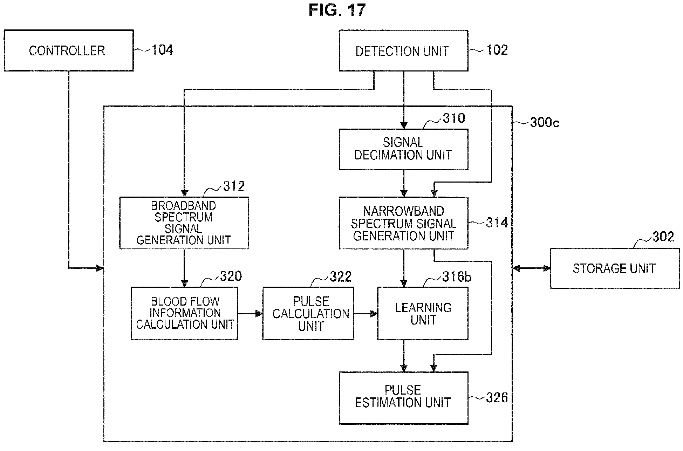

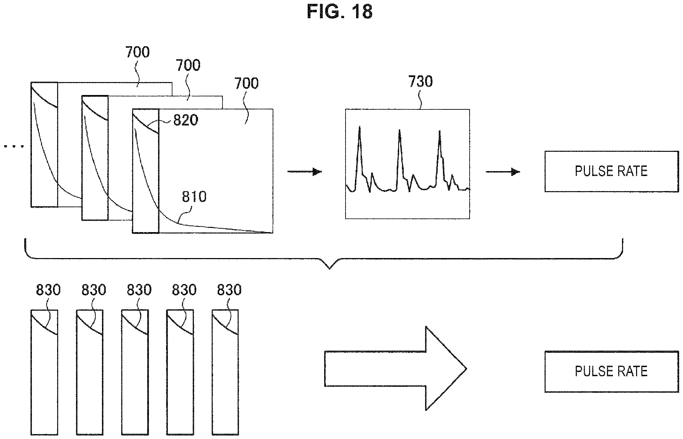

In the embodiment, each functional unit included in the processor 300c is different from that of the processors 300 and 300b according to the first and second embodiments. Hereinafter, a configuration of the processor 300c according to the embodiment will be described with reference to FIGS. 17 and 18. FIG. 17 is a block diagram illustrating a functional configuration of the processor 300c according to the embodiment. FIG. 18 is an explanatory diagram illustrating an information processing method according to the embodiment.

As illustrated in FIG. 17, the processor 300c mainly includes the inter-signal decimation unit 310, the broadband spectrum signal generation unit 312, the narrowband spectrum signal generation unit 314, a learning unit 316b, the blood flow information calculation unit 320, the pulse calculation unit 322, and a pulse estimation unit 326. That is, in the embodiment, the learning unit 316b and the pulse estimation unit 326 are different from those of the second embodiment. Accordingly, the description of the common functional units to those of the second embodiment will be omitted and only an association relation between the functional units, the learning unit 316b, and the pulse estimation unit 326 will be described.

### (Association relation between functional units in processor 300c according to third embodiment)

In the third embodiment, a calculation result of the blood flow information calculation unit 320 is output to the pulse calculation unit 322. A calculation result of the pulse calculation unit 322 is output to the learning unit 316b. A calculation result of the narrowband spectrum signal generation unit 314 is output to the learning unit 316b and the pulse estimation unit 326. A learning result of the learning unit 316b is used when the pulse estimation unit 326 performs estimation via the storage unit 302.

### (Learning unit 316b)

The learning unit 316b performs machine learning using a pulse rate output from the pulse calculation unit 322 and the second power spectrum 820 output from the narrowband spectrum signal generation unit 314 in the first operation. Then, relation information between the pulse rate and the second power spectrum 820 obtained through the machine learning in the learning unit 316b is stored in the storage unit 302 to be used in the pulse estimation unit 326 to be described below. Note that the pulse rate output to the learning unit 316b is obtained by processing the plurality of first detection signals in which a folding noise component is small to the extent that an adverse influence of the folding noise component is negligible. More specifically, as illustrated in the upper stage of FIG. 18, the learning unit 316b acquires a pulse rate from a pulse rate waveform 730 acquired from the first power spectrum 810. Further, the learning unit 316b acquires the second power spectrum 820 forming the power spectrum pair 700 along with the first power spectrum 810. Then, the learning unit 316b learns a relation between the pulse rate and the second power spectrum 820. In other words, the learning unit 316b learns a relation between the second power spectrum 820 on which the folding noise component is superimposed and the pulse rate on which there is no influence of the folding noise component. Note that in order for the learning unit 316b to perform the learning efficiently, it is preferably to input the plurality of second power spectra 820 with different time ranges to the learning unit 316b throughout a period in which a difference in the time range is longer than a pulse interval of the pulse. Note that the learning unit 316b can also perform the learning by inputting a heartbeat rate measured using another measurer from the outside of the processor 300c to the learning unit 316b as an alternative of the information from the pulse calculation unit 322. In this case, the estimation by the pulse estimation unit is a heartbeat rate rather than a pulse rate in theory of machine learning, but is equal to estimation of a pulse rate in practice because of estimation on the basis of blood flow information. By realizing such modification, an additional measurer is necessary, but it is possible to further improve accuracy of a supervised signal used for the learning.

### (Pulse estimation unit 326)

The pulse estimation unit 326 estimates a pulse rate from the third power spectrum 830 on which a folding noise component is superimposed on the basis of the relation information obtained by the learning unit 316b in the second operation. The pulse rate obtained by the pulse estimation unit 326 may be output to the storage unit 302 or may be output to the above-described information posting apparatus (not illustrated). More specifically, the pulse estimation unit 326 estimates a pulse rate illustrated in the lower stage of FIG. 18 on the basis of the relation learning (see the upper stage of FIG. 18) from each third power spectrum 830 illustrated in the lower stage of FIG. 18.

### <6.2 Information processing method according to third embodiment>

Next, an information processing method according to the embodiment will be described. The information processing method according to the embodiment can be divided into a first operation and a second operation as in the first embodiment. First, the first operation of the information processing method according to the embodiment will be described with reference to FIG. 11 which is a flowchart of the first operation according to the first embodiment.

The first operation according to the embodiment is different from that of the first embodiment in that blood flow information and a pulse rate (first pulse rate) are calculated in addition to the generation of the power spectrum in step S105 illustrated in FIG. 11. Further, the first operation according to the embodiment is different from that of the first embodiment in that in step S107 illustrated in FIG. 11, the relation between the first power spectrum 810 and the second power spectrum 820 is not learned, and the relation between the pulse rate and the second power spectrum 820 is learned.

Next, the second operation of the information processing method according to the embodiment will be described with reference to FIG. 12 which is the flowchart of the second operation according to the first embodiment. The second operation according to the embodiment is different from that of the first embodiment in that step S205 and step S207 illustrated in FIG. 12 are skipped and the pulse rate (second pulse rate) is estimated from the third power spectrum 830 instead of calculating the pulse rate from the blood flow information in step S209 illustrated in FIG. 12.

As described above, in the embodiment, the third power spectrum 830 on which the folding noise component is superimposed since the sampling frequency is lowered is detected as in the first embodiment. Accordingly, in the embodiment, the pulse rate obtained from the first power spectrum 810 in which the folding noise component is small to the extent that the adverse influence of the folding noise component is negligible is acquired. Further, the relation information is acquired by performing the machine learning of the relation between the pulse rate and the power spectrum 820 on which the folding noise component is superimposed and which corresponds to the first power spectrum 810. Then, in the embodiment, on the basis of the relation information, the pulse rate on which there is no influence of the folding noise component is estimated from the foregoing power spectrum 830. Accordingly, in the embodiment, it is possible to obtain the accurate blood flow information from the power spectrum 830 on which the folding noise component is superimposed since the sampling frequency is lowered, while suppressing the power consumption of the measurement module 10 by lowering the sampling frequency. Furthermore, in the embodiment, it is possible to reduce a processing amount in the second operation because of the direct estimation of the pulse rate.

Note that embodiments of the present disclosure are not limited to the above-described first to third embodiments. Another piece of blood flow information or information such as a function with a specific relation such as proportion or inverse proportion to specific blood flow information may be estimated and the present disclosure is not particularly limited. For example, in an embodiment of the present disclosure, a relation between blood flow information or the like (first blood flow information) obtained at the high first sampling frequency and blood flow information (second blood flow information) obtained at the low second sampling frequency is learned (a relation between two different kinds of blood flow information or the like is learned). In this case, on the basis of the learned relation information, blood flow information or the like (fourth blood flow information) which is obtained at the high first sampling frequency and in which a folding noise component is small to the extent that the adverse influence of the folding noise component is negligible is estimated from blood flow information or the like (third blood flow information) obtained at the low second sampling frequency in another blood flow measurement. That is, in the embodiment of the present disclosure, another kind of blood flow information or the like corresponding to one kind of blood flow information or the like is estimated from the one kind of blood flow information or the like. In addition, for example, in the embodiment of the present disclosure, a relation between blood flow information or the like on which there is no influence of noise (noise is not superimposed) and blood flow information on which there is an influence of noise (noise is superimposed) may be learned. In this case, on the basis of the learning, blood flow information or the like on which there is no influence of noise is estimated from blood flow information or the like on which there is an influence of noise and which is obtained in another flood flow measurement.

### <<7. Fourth embodiment>>

To improve accuracy of the estimation performed in the above-described first to third embodiments, it is preferable to use the detection signal or the blood flow information on which other noise components are not superimposed as a matter of course. As an instance in which other noise components are removed, for example, an instance in which an exercise in which the measured person shakes his or her arm when blood flow measurement is performed to obtain a pulse rate is performed is known. In this instance, since acceleration occurring in the exercise is used to change blood flow and noise components are caused to arise, the noise components causing a change in the blood flow are cancelled using information of an accelerometer. However, for example, when a finger moves at the time of measurement of a signal in the arm, noise components caused due to the movement may be superimposed on a detection signal or the like in some cases. More specifically, a blood flow velocity of the arm of the measured person is modulated due to a physical change in a blood flow passage before the arm caused due to the movement of the finger at the time of blood flow measurement, the blood flow velocity is mixed with a variation by heartbeat, and an accurate heartbeat rate (heartbeat waveform) may not be acquired in some cases. However, in this case, since acceleration of the arm is scarcely changed, an obtainable effect is small when noise components are cancelled using the above-described accelerometer. Accordingly, in a fourth embodiment, an embodiment in which accurate blood flow information can be obtained by removing noise components caused due to a body motion of the measured person from a detection signal, blood flow information, or the like will be proposed. For example, in the embodiment, by combining the above-described first to third embodiments, it is possible to further improve accuracy of the learning or the estimation in each embodiment.

In particular, there are various body motions of the measured person corresponding to the above-described example. For example, in a case in which the measurement module 10 is considered to be mounted on a wrist of the measured person, an exercise in which an arm on which the measurement module 10 is mounted is shaken, a typing exercise of fingertips, and the like can be exemplified. Then, blood flow is variously influenced due to a difference in the body motion. Specifically, for example, in a case in which a body motion is an exercise in which an arm is shaken, a blood flow velocity in an artery of the arm is modulated due to the body motion and blood flow information detected from the artery contains noise components caused due to the body motion. In the present disclosure, since a measurement part is not a broad region, the measurement part is moved as one substantially integrated part and acceleration by the exercise is almost uniform. On the other hand, a resistant constant received from a blood vessel serving as a flow passage differs depending on a kind of blood vessel in which there is blood. Since an actually caused change in an exercise of particles in a blood vessel is made by a resultant force of both parties, a tendency of the change differs for each kind of blood vessel in which there are the particles. In addition, in a case in which a body motion is a typing exercise, propagation of an influence of deformation of fingertips differs for each kind of blood vessel. Therefore, a tendency of exercises of particles also differs for each kind of blood vessel. That is, depending on a body motion, an influence of the body motion differs for each kind of blood vessel in the body.

Incidentally, in a blood flow information measurement method used in the embodiment, as described above, blood flow information in blood vessels in a biological tissue within a range in which radiated light arrives can be acquired at one time. More specifically, in the blood flow information measurement method used in the embodiment, blood flow information regarding not only capillaries near the surface of skin of the measured person but also inner arterioles or the like can be acquired at one time. Accordingly, a detection signal detected in the embodiment includes a plurality of signal components obtained from the various blood vessels. Moreover, in a method of obtaining a relative particle density in blood flow in a predetermined velocity range used in the embodiment, blood flow velocities of flowing blood differ for each kind of blood vessel (for example, about several to tens of mm/sec in arterioles and about hundreds of µm/sec in capillaries). Therefore, the foregoing detection signal is processed in a range suitable for a blood flow velocity range for each kind of blood vessel. As a result, in the method, it is possible to obtain an independent signal component for each kind of blood vessel.

Accordingly, in the embodiment, noise components caused due to a body motion are calculated using a plurality of independent signal components obtained by processing power spectra obtained from one detection signal in a plurality of ranges and the calculated noise components are removed from the detection signal. In particular, for example, in a case in which noise components caused due to a body motion are superimposed as an irregular signal on one of the plurality of signal components, the signal component on which the irregular signal is superimposed is compared to a signal component on which the irregular signal is not superimposed. Then, by extracting the irregular signal on the basis of the comparison, the noise components can be calculated. In addition, for example, in a case in which noise components caused due to a body motion are superimposed as an irregular signal at a specific ratio in a plurality of signal components among the plurality of signal components, the noise components can be calculated by comparing signal components on which the irregular signal is superimposed and extracting the common irregular signal. Further, as in the above-described first to third embodiments, a relation between each signal component and noise components caused to a body motion may be learned and the noise components may be estimated from a plurality of signal components included in detection signals obtained from other blood flow measurements on the basis of the relation information obtained through the learning. In addition, in a case in which an acceleration sensor (not illustrated) is included in the information processing system according to the embodiment, accuracy of the calculation of the noise components may be improved by additionally using a detection result obtained by the acceleration sensor.

According to the embodiment, the accurate blood flow information can be obtained by using the detection signal from which the noise components are removed. Hereinafter, this embodiment will be described as the fourth embodiment. Note that since the configurations of the information processing system 1, the measurement module 10, and the information processing apparatus 30 according to the embodiment have been described above, the description thereof will be omitted herein. Further, in the embodiment, the information processing system 1 may include an acceleration sensor or the like, as described above. The acceleration sensor is contained in, for example, the measurement module 10. Alternatively, apart from the measurement module 10, one acceleration sensor or a plurality of acceleration sensors are mounted at predetermined spots of the body of the measured person to detect a motion of a part of the body of the measured person.

### <7.1 Configuration of processor 300d according to fourth embodiment>

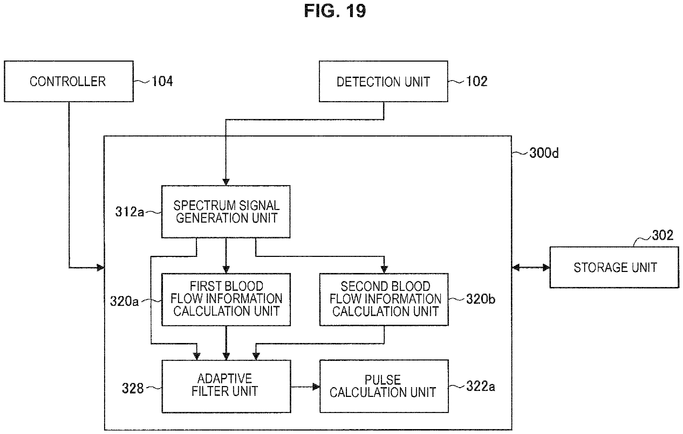

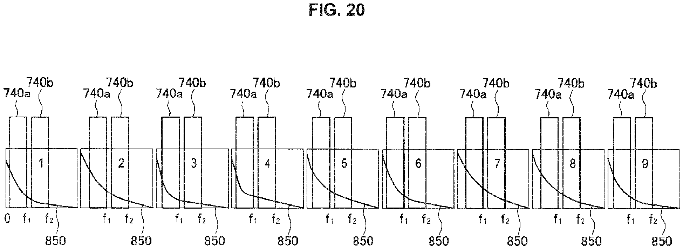

In the embodiment, each functional unit included in the processor 300d is different from that of the processor 300 according to the first embodiment. Hereinafter, a configuration of the processor 300d according to the embodiment will be described with reference to FIGS. 19 and 20. FIG. 19 is a block diagram illustrating a functional configuration of the processor 300d according to the embodiment. FIG. 20 is an explanatory diagram illustrating an information processing method according to the embodiment. As illustrated in FIG. 19, the processor 300d mainly includes a spectrum signal generation unit 312a, a first blood flow information calculation unit 320a, a second blood flow information calculation unit 320b, a pulse calculation unit 322a, and an adaptive filter unit 328. Hereinafter, each functional unit included in the processor 300d will be described.

### (Spectrum signal generation unit 312a)

The spectrum signal generation unit 312a performs a process on a detection signal detected by the detection unit 102 of the measurement module 10 to generate power spectra and outputs the power spectra to the first blood flow information calculation unit 320a, the second blood flow information calculation unit 320b, and the adaptive filter unit 328 to be described below.

### (First blood flow information calculation unit 320a and second blood flow information calculation unit 320b)

The first blood flow information calculation unit 320a and the second blood flow information calculation unit 320b perform processes on the power spectra output from the spectrum signal generation unit 312a in mutually different frequency ranges to obtain mutually different signal components. In particular, as illustrated in FIG. 20, the first blood flow information calculation unit 320a and the second blood flow information calculation unit 320b perform processes on the plurality of power spectra 850 output from the spectrum signal generation unit 312a. More specifically, the first blood flow information calculation unit 320a performs a process on each power spectrum 850 in a first frequency range 740a to acquire a first signal component. In addition, the second blood flow information calculation unit 320b performs a process on each power spectrum 850 in a second frequency range 740b which is a different range from the first frequency range 740a to acquire a second signal component. Then, the acquired first and second signal components are output to the adaptive filter unit 328 to be described below.

Note that the first frequency range 740a and the second frequency range 740b are set to ranges in accordance with a range of blood flow velocity of each kind of blood vessel, as described above. Alternatively, the first frequency range 740a and the second frequency range 740b may be set to any ranges rather than the ranges in accordance with the range of the blood flow velocity of each kind of blood vessel. In addition, the number of blood flow information calculation units according to the embodiment is not limited to two, as illustrated in FIG. 19 and three or more blood flow information calculation units may be installed. In addition, in order to improve accuracy of the calculation of noise components caused due to a body motion, it is preferable to use independent signal components as many as possible, and thus it is preferable to provide many blood flow information calculation units 320.

### (Pulse calculation unit 322a)

The pulse calculation unit 322a calculates a pulse rate using blood flow information from which the noise components caused due to the body motion are removed by the adaptive filter unit 328 to be described below.

### (Adaptive filter unit 328)

The adaptive filter unit 328 calculates the noise components caused due to the body motion using the power spectra output from the spectrum signal generation unit 312a and two independent first and second signal components obtained from the first blood flow information calculation unit 320a and the second blood flow information calculation unit 320b. Further, the adaptive filter unit 328 removes the calculated noise components from the first and second signal components from the first blood flow information calculation unit 320a and the second blood flow information calculation unit 320b and acquires combination values so that an adverse influence of the noise components and the combination is small. Then, the adaptive filter unit 328 outputs the acquired values to the above-described pulse calculation unit 322c.

### <7.2 Information processing method according to fourth embodiment>

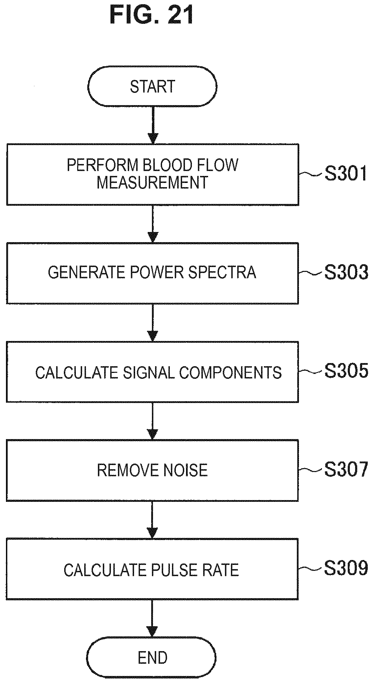

Next, an information processing method according to the embodiment will be described with reference to FIG. 21. FIG. 21 is a diagram illustrating of a flowchart of the information processing method according to the embodiment. As illustrated in FIG. 21, the information processing method according to the embodiment includes step S301 to step S309. Hereinafter, each step of the information processing method according to the embodiment will be described.

First, before the information processing method according to the embodiment starts, for example, as illustrated in FIG. 4, the above-described measurement module 10 is mounted on a wrist or the like of the measured person.

### (Step S301)

When it is detected that the measurement module 10 is mounted on a part of the body of the measured person, or the like, the measurement module 10 starts the blood flow measurement. Then, the information processing apparatus 30 acquires a detection signal. Note that the blood flow measurement may be performed a plurality of times by repeating step S301.

### (Step S303)

The information processing apparatus 30 performs a process on the detection signal detected in step S301 to generate the power spectra 850. Note that in a case in which the blood flow measurement is performed the plurality of times by repeating the above-described step S301, step S303 is repeated the plurality of times.

### (Step S305)

The information processing apparatus 30 performs a process on the power spectra 850 generated in step S303 to acquire a plurality of signal components.

### (Step S307)

The information processing apparatus 30 calculates noise components caused due to a body motion using the power spectra 850 acquired in step S303 and the plurality of signal components acquired in step S305 and removes the noise components calculated from the signal components generated in step S305.

### (Step S309)

The information processing apparatus 30 calculates a pulse rate using the signal components from which the noise components caused due to the body motion are removed and which are acquired in step S307.

As described above, in the embodiment, the noise components caused due to the body motion are calculated using the plurality of independent signal components obtained by processing one detection signal in the plurality of ranges, and the calculated noise components are removed from the detection signal. Accordingly, according to the embodiment, by using the detection signal from which the noise components caused due to the body motion are removed, it is possible to obtain the accurate blood flow information. Further, according to the embodiment, by processing one detection signal in the plurality of ranges, it is possible to obtain the plurality of independent signal components and avoid providing the plurality of radiation units 100 and the plurality of detection units 102. As a result, since the plurality of radiation units 100 and the plurality of detection units 102 are not provided, it is possible to suppress an increase in the size of the measurement module 10 and manufacturing cost of the measurement module 10. Furthermore, by combining the embodiment with the above-described first to third embodiments, it is possible to further improve accuracy of the learning or the estimation in each embodiment. In addition, in the embodiment, for example, the measurement modules 10 may be mounted on both arms of the measured person so that learning can be performed using a detection signal (blood flow information) obtained from one stopping arm and a detection signal (blood flow information) obtained from the other arm affected by disturbance caused due to a body motion as a supervised signal and an input signal, respectively. In this case, the detection signal (blood flow information) of the other arm which is not affected by the disturbance caused due to the body motion, that is, on which noise components are not superimposed, can be estimated in accordance with the relation information obtained through the learning. Then, for example, by applying the estimated detection signal on which the noise components caused due to the body motion are not superimposed as the supervised signal to the second or third embodiment, it is possible to improve accuracy of the learning or the estimation in the second or third embodiment.

Note that in the above-described embodiment, the plurality of independent signal components have been obtained by processing the power spectra in the plurality of ranges, as described above, but the embodiment is not limited thereto. For example, by processing the power spectra to acquire the blood flow information and processing blood flow information acquired under a predetermined condition, the plurality of independent signal components included in the blood flow information may be obtained.

In addition, in the embodiment, the finally obtained blood flow information (for example, a pulse rate) may be analyzed, the noise components caused due to the body motion and superimposed on the blood flow information may be partitioned, and calculation or removal of the noise may be dynamically controlled on the basis of the result. In particular, in the embodiment, for example, as the control, a frequency range used at the time of acquisition of the signal components is caused to be dynamically changed in accordance with the calculated noise components. By performing the feedback control, it is also possible to optimize the frequency range or the like and decrease or remove the noise components included in the finally obtained blood flow information.

### <<8. Hardware configuration>>

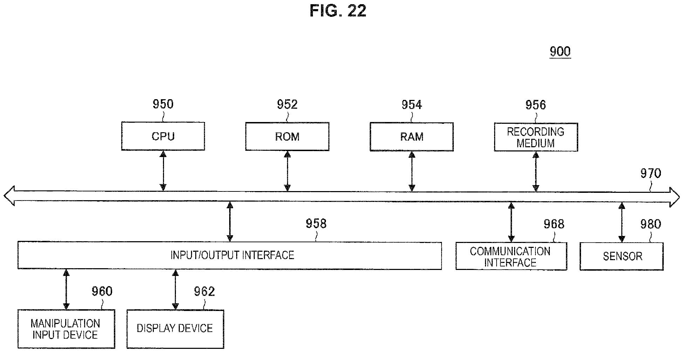

FIG. 22 is an explanatory diagram illustrating an example of a hardware configuration of an information processing apparatus 900 according to an embodiment of the present disclosure. In FIG. 22, the information processing apparatus 900 is an example of a hardware configuration of the above-described information processing apparatus 30.

The information processing apparatus 900 includes, for example, a CPU 950, a ROM 952, a RAM 954, a recording medium 956, an input/output interface 958, and a manipulation input device 960. Further, the information processing apparatus 900 includes a display device 962, a communication interface 968, and a sensor 980. Further, in the information processing apparatus 900, for example, the respective components are connected by a bus 970 serving as a data transmission path.

### (CPU 950)

The CPU 950 includes, for example, one processor or two or more processors configured as an arithmetic circuit such as a CPU, various processing circuits, and the like, functions as a controller (not illustrated) that controls the entire information processing apparatus 900, and functions as the processor 300 that processes a detection result.

### (ROM 952 and RAM 954)

The ROM 952 stores a program, control data such as operation parameters, and the like used by the CPU 950. The RAM 954 temporarily stores, for example, a program executed by the CPU 950. The ROM 952 and the RAM 954 achieve, for example, the function of the above-described storage unit 302 in the information processing apparatus 900.

### (Recording medium 956)

The recording medium 956 functions as the storage unit 302, and stores, for example, data related to the information processing method according to the present embodiment and various data such as various kinds of applications. Here, examples of the recording medium 956 include a magnetic recording medium such as a hard disk and a non-volatile memory such as a flash memory. Further, the recording medium 956 may be removable from the information processing apparatus 900.

### (Input/output interface 958, manipulation input device 960, and display device 962)

The input/output interface 958 connects, for example, the manipulation input device 960, the display device 962, and the like. Examples of the input/output interface 958 include a universal serial bus (USB) terminal, a digital visual interface (DVI) terminal, a high-definition multimedia interface (HDMI) (registered trademark) terminal, and various kinds of processing circuits.

The manipulation input device 960 functions as a manipulating unit (not illustrated), is installed in, for example, the information processing apparatus 900, and is connected with the input/output interface 958 in the information processing apparatus 900. Examples of the manipulation input device 960 include a button, a directional key, a rotary selector such as a jog dial, a touch panel, and a combination thereof.

The display device 962 functions as the information presenting section (not shown) including the display apparatus, and is installed, for example, in the information processing apparatus 900 and connected with the input/output interface 958 in the information processing apparatus 900. Examples of the display device 962 include a liquid crystal display, an organic electro-luminescence (EL) display, and the like.

Further, it goes without saying that the input/output interface 958 can also be connected with an external device such as a manipulation input device (for example, a keyboard, a mouse, or the like) outside the information processing apparatus 900 or an external display device.

### (Communication interface 968)

A communication interface 968 is a communication unit installed in the information processing apparatus 900 and functions as a communication section (not illustrated) for performing wireless or wired communication with an external apparatus such as a server via a network (or directly). Here, examples of the communication interface 968 include a communication antenna and a radio frequency (RF) circuit (wireless communication), an IEEE 802.15.1 port and a transceiving circuit (wireless communication), an IEEE 802.11 port and a transceiving circuit (wireless communication), and a local area network (LAN) terminal and a transceiving circuit (wired communication).

### (Sensor 980)

The sensor 980 is a sensor that functions as the measurement module 10 and detects blood flow signals in accordance with an arbitrary scheme capable of detecting the blood flow signals or the like caused by the blood flow. The sensor 980 includes, for example, the radiation unit 100 that emits light and the detection unit 102 that generates a signal in response to received light. The radiation unit 100 includes, as described earlier, for example, one or more light sources such as a laser. Further, the detection unit 102 also includes, for example, a photodiode, an amplifier circuit, a filter circuit, and an analog-to-digital converter.

Further, the sensor 980 may include, for example, one sensor or two or more sensors capable of detecting a motion of the body of a measured person, such as an acceleration sensor, a gyro sensor, or the like. Further, the sensor 980 may include a pressure sensor or the like capable of detecting a mounting state of the above-described measurement module 10. Note that a sensor included in the sensor 980 is not limited to the above-described example.

Note that a hardware configuration of the information processing apparatus 900 is not limited to the configuration illustrated in FIG. 22. For example, in this case in which the information processing apparatus 900 communicates with an external apparatus or the like via an external communication device connected thereto or in a case in which the information processing apparatus 900 is configured to perform standalone processing, the information processing apparatus 900 may not include the communication interface 968. Further, the communication interface 968 may have a configuration capable of communicating with one or more external apparatuses in accordance with a plurality of communication schemes. Further, the information processing apparatus 900 can also have, for example, a configuration in which the recording medium 956, the manipulation input device 960, the display device 962, or the like is not included.

While the information processing apparatus has been described as the present invention, the present embodiment is not limited to such an embodiment. The present embodiment can be applied to various devices capable of performing processing related to the information processing method according to the present embodiment such as a communication apparatus such as a cellular phone or the like.

Further, the information processing apparatus according to the present embodiment may be applied to a system including a plurality of apparatuses based on a connection to a network (or communication between respective apparatuses) as in cloud computing or the like. **In** other words, the information processing apparatus according to the present embodiment can also be realized as, for example, an information processing system which performs processing related to the information processing method according to the present embodiment through a plurality of apparatuses.

The example of the hardware configuration of the information processing apparatus 900 has been described above. Each of the components may be constituted using a general-purpose member or may be constituted by hardware specialized for the function of each component. Such a configuration can be appropriately changed in accordance with a technical level at the time of implementation.

### <<9. Supplement>>

Further, the embodiments of the present disclosures described above may include, for example, a program causing a computer to function as the information processing apparatus according to the present embodiment and a non-transitory tangible medium having a program recorded therein. Further, the program may be distributed via a communication line such as the Internet (including wireless communication).

Further, the steps in the process of each of the above-described embodiments may not necessarily be processed in the described order. For example, the order of the steps may be appropriately modified so that the steps are processed. Further, some of the steps may be processed in parallel or individually instead of being processed chronologically. Moreover, a processing method of the steps may not necessarily be processed in accordance with the described method and may be processed in accordance with another method by other functional units, for example.

The preferred embodiment(s) of the present disclosure has/have been described above with reference to the accompanying drawings, whilst the present disclosure is not limited to the above examples. A person skilled in the art may find various alterations and modifications within the scope of the appended claims, and it should be understood that they will naturally come under the technical scope of the present disclosure.

Further, the effects described in this specification are merely illustrative or exemplified effects, and are not limitative. That is, with or in the place of the above effects, the technology according to the present disclosure may achieve other effects that are clear to those skilled in the art from the description of this specification.

### Reference Signs List

1 information processing system
10 measurement module
30 information processing apparatus
70 stationary tissue
72 scattering substance
100 radiation unit
102, 102a, 102b detection unit
104 controller
110 band unit
112 control unit
114 measurement unit
116 adhesive layer
118 fixing unit
300, 300a, 300b, 300c, 300d processor
302 storage unit
310 inter-signal decimation unit
312, 312a, 314 spectrum signal generation unit
316, 316a, 316b learning unit
318 spectrum signal estimation unit
320, 320a, 320b blood flow information calculation unit
322, 322a pulse calculation unit
324 blood flow information estimation unit
326 pulse estimation unit
328 adaptive filter unit
500 window
502 detection signal
600, 606, 810, 820, 830, 840, 850 power spectrum
602, 604 folding noise component
700 power spectrum pair
710 blood flow velocity distribution
720 particle density distribution
730 pulse waveform
740a, 740b frequency range
900 information processing apparatus
950 CPU
952 ROM
954 RAM
956 recording medium
958 input/output interface
960 manipulation input device
962 display device
968 communication interface
970 bus
980 sensor

## Claims

1. An information processing apparatus (30) comprising:
an estimation unit (318) configured to estimate, on a basis of relation information, a fourth power spectrum (840) corresponding to a first sampling frequency from a third power spectrum (830) obtained through a third blood flow measurement at a second sampling frequency lower than the first sampling frequency, wherein the relation information is information indicating a relation between a first power spectrum (810) obtained through a first blood flow measurement at the first sampling frequency and a second power spectrum (820) obtained through a second blood flow measurement at the second sampling frequency;
a broadband spectrum signal generation unit (312) configured to generate the first power spectrum (810) from a first blood flow signal obtained through the first blood flow measurement;
a narrowband spectrum signal generation unit (314) configured to generate the third power spectrum (830) from a third blood flow signal obtained through the third blood flow measurement and the second power spectrum (820) from a second blood flow signal obtained through the second blood flow measurement;
a learning unit (316) configured to perform machine learning using the first power spectrum (810) and the second power spectrum (820) to learn the relation information;
a storage unit (302) configured to store the relation information; and
a blood flow information calculation unit (320) configured to calculate a blood flow velocity or relative densities of particles in blood vessels within a predetermined velocity range using the fourth power spectrum (840) estimated by the estimation unit (318).

2. The information processing apparatus (30) according to claim 1, further comprising:
a measurement unit (114) configured to be mounted on a part of a body of a measured person and perform the blood flow measurement on the measured person.

3. The information processing apparatus (30) according to claim 2, wherein the measurement unit (114) includes
a radiation unit (100) configured to radiate light to the part of the body of the measured person,
a first detection unit (102a) configured to detect light from the part of the body of the measured person and to acquire the first blood flow signal,
a second detection unit (102b) configured to detect light from the part of the body of the measured person and to acquire the second blood flow signal and the third blood flow signal, and
a controller (104) configured to control a sampling frequency that decides a radiation timing of the radiation unit (100) and a reading timing for reading a detection result of the detection unit (102).

4. The information processing apparatus (30) according to claim 3,
wherein the radiation unit (100) radiates light with a predetermined frequency, and
the detection unit (102) detects interference light between light with the predetermined frequency and light with a frequency different from the predetermined frequency.

5. A computer-implemented method comprising:
estimating, on a basis of relation information, a fourth power spectrum (840) corresponding to a first sampling frequency from a third power spectrum (830) obtained through a third blood flow measurement at a second sampling frequency lower than the first sampling frequency, wherein the relation information is information indicating a relation between a first power spectrum (810) obtained through a first blood flow measurement at the first sampling frequency and a second power spectrum (820) obtained through a second blood flow measurement at the second sampling frequency;
generating the first power spectrum (810) from a first blood flow signal obtained through the first blood flow measurement;
generating the third power spectrum (830) from a third blood flow signal obtained through the third blood flow measurement and the second power spectrum (820) from a second blood flow signal obtained through the second blood flow measurement;
performing machine learning using the first power spectrum (810) and the second power spectrum (820) to learn the relation information;
storing the relation information; and
calculating a blood flow velocity or relative densities of particles in blood vessels within a predetermined velocity range using the estimated fourth power spectrum (840).

6. A computer program comprising instructions which, when the program is executed by a computer, cause the computer to carry out the computer-implemented method of claim 5.

## Patentansprüche

1. Informationsverarbeitungseinrichtung (30), umfassend:
eine Abschätzeinheit (318), die konfiguriert ist, um, auf einer Grundlage von Relationsinformationen, ein viertes Leistungsspektrum (840), das einer ersten Abtastfrequenz entspricht, aus einem dritten Leistungsspektrum (830) abzuschätzen, das durch eine dritte Blutflussmessung bei einer zweiten Abtastfrequenz erhalten wird, die niedriger als die erste Abtastfrequenz ist, wobei die Relationsinformationen Informationen sind, die eine Relation zwischen einem ersten Leistungsspektrum (810), das durch eine erste Blutflussmessung bei der ersten Abtastfrequenz erhalten wird, und einem zweiten Leistungsspektrum (820) angeben, das durch eine zweite Blutflussmessung bei der zweiten Abtastfrequenz erhalten wird;
eine Breitbandspektrumsignalerzeugungseinheit (312), die konfiguriert ist, um das erste Leistungsspektrum (810) aus einem ersten Blutflusssignal zu erzeugen, das durch die erste Blutflussmessung erhalten wird;
eine Schmalbandspektrumsignalerzeugungseinheit (314), die konfiguriert ist, um das dritte Leistungsspektrum (830) aus einem dritten Blutflusssignal zu erzeugen, das durch die dritte Blutflussmessung erhalten wird, und das zweite Leistungsspektrum (820) aus einem zweiten Blutflusssignal zu erzeugen, das durch die zweite Blutflussmessung erhalten wird;
eine Lerneinheit (316), die konfiguriert ist, um maschinelles Lernen unter Verwendung des ersten Leistungsspektrums (810) und des zweiten Leistungsspektrums (820) durchzuführen, um die Relationsinformationen zu erlernen;
eine Speichereinheit (302), die konfiguriert ist, um die Relationsinformationen zu speichern; und
eine Blutflussinformationsberechnungseinheit (320), die konfiguriert ist, um eine Blutflussgeschwindigkeit oder relative Dichten von Partikeln in Blutgefäßen innerhalb eines zuvor bestimmten Geschwindigkeitsbereichs unter Verwendung des vierten Leistungsspektrums (840) zu berechnen, das durch die Abschätzeinheit (318) abgeschätzt wird.

2. Informationsverarbeitungseinrichtung (30) nach Anspruch 1, ferner umfassend:
eine Messeinheit (114), die konfiguriert ist, um an einem Körperteil einer gemessenen Person montiert zu werden und die Blutflussmessung an der gemessenen Person durchzuführen.

3. Informationsverarbeitungseinrichtung (30) nach Anspruch 2, wobei die Messeinheit (114) einschließt
eine Strahlungseinheit (100), die konfiguriert ist, um Licht auf das Körperteil der gemessenen Person auszustrahlen,
eine erste Detektionseinheit (102a), die konfiguriert ist, um Licht von dem Körperteil der gemessenen Person zu detektieren und das erste Blutflusssignal zu erfassen,
eine zweite Detektionseinheit (102b), die konfiguriert ist, um Licht von dem Körperteil der gemessenen Person zu detektieren und das zweite Blutflusssignal und das dritte Blutflusssignal zu erfassen, und
eine Steuerung (104), die konfiguriert ist, um eine Abtastfrequenz zu steuern, die einen Strahlungszeitpunkt der Strahlungseinheit (100) und einen Lesezeitpunkt zum Lesen eines Detektionsergebnisses der Detektionseinheit (102) entscheidet.

4. Informationsverarbeitungseinrichtung (30) nach Anspruch 3,
wobei die Strahlungseinheit (100) Licht mit einer zuvor bestimmten Frequenz ausstrahlt, und
die Detektionseinheit (102) Interferenzlicht zwischen Licht mit der zuvor bestimmten Frequenz und Licht mit einer Frequenz detektiert, die sich von der zuvor bestimmten Frequenz unterscheidet.

5. Computerimplementiertes Verfahren, umfassend:
Abschätzen, auf einer Grundlage von Relationsinformationen, eines vierten Leistungsspektrums (840), das einer ersten Abtastfrequenz entspricht, aus einem dritten Leistungsspektrum (830), das durch eine dritte Blutflussmessung bei einer zweiten Abtastfrequenz erhalten wird, die niedriger als die erste Abtastfrequenz ist, wobei die Relationsinformationen Informationen sind, die eine Relation zwischen einem ersten Leistungsspektrum (810), das durch eine erste Blutflussmessung bei der ersten Abtastfrequenz erhalten wird, und einem zweiten Leistungsspektrum (820) angeben, das durch eine zweite Blutflussmessung bei der zweiten Abtastfrequenz erhalten wird;
Erzeugen des ersten Leistungsspektrums (810) aus einem ersten Blutflusssignal, das durch die erste Blutflussmessung erhalten wird;
Erzeugen des dritten Leistungsspektrums (830) aus einem dritten Blutflusssignal, das durch die dritte Blutflussmessung erhalten wird, und des zweiten Leistungsspektrums (820) aus einem zweiten Blutflusssignal, das durch die zweite Blutflussmessung erhalten wird;
Durchführen eines maschinellen Lernens unter Verwendung des ersten Leistungsspektrums (810) und des zweiten Leistungsspektrums (820), um die Relationsinformationen zu erlernen;
Speichern der Relationsinformationen; und
Berechnen einer Blutflussgeschwindigkeit oder relativen Dichte von Partikeln in Blutgefäßen innerhalb eines zuvor bestimmten Geschwindigkeitsbereichs unter Verwendung des abgeschätzten vierten Leistungsspektrums (840).

6. Computerprogramm, umfassend Anweisungen, die, wenn das Programm durch einen Computer ausgeführt wird, den Computer veranlassen, das computerimplementierte Verfahren nach Anspruch 5 vorzunehmen.

## Revendications

1. Appareil de traitement d'informations (30) comprenant :
une unité d'estimation (318) configurée pour estimer, en fonction d'informations de relation, un quatrième spectre de puissance (840) correspondant à une première fréquence d'échantillonnage à partir d'un troisième spectre de puissance (830) obtenu par le biais d'une troisième mesure de flux sanguin à une seconde fréquence d'échantillonnage inférieure à la première fréquence d'échantillonnage, dans lequel les informations de relation sont des informations indiquant une relation entre un premier spectre de puissance (810) obtenu par le biais d'une première mesure de flux sanguin à la première fréquence d'échantillonnage et un deuxième spectre de puissance (820) obtenu par le biais d'une deuxième mesure de flux sanguin à la seconde fréquence d'échantillonnage ;
une unité de génération de signal de spectre à large bande (312) configurée pour générer le premier spectre de puissance (810) à partir d'un premier signal de flux sanguin obtenu par le biais de la première mesure de flux sanguin ;
une unité de génération de signal de spectre à bande étroite (314) configurée pour générer le troisième spectre de puissance (830) à partir d'un troisième signal de flux sanguin obtenu par le biais de la troisième mesure de flux sanguin et du deuxième spectre de puissance (820) à partir d'un deuxième signal de flux sanguin obtenu par le biais de la deuxième mesure de flux sanguin ;
une unité d'apprentissage (316) configurée pour mettre en œuvre un apprentissage automatique à l'aide du premier spectre de puissance (810) et du deuxième spectre de puissance (820) pour apprendre les informations de relation ;
une unité de stockage (302) configurée pour stocker les informations de relation ; et
une unité de calcul d'informations de flux sanguin (320) configurée pour calculer une vitesse de flux sanguin ou des densités relatives de particules dans des vaisseaux sanguins au sein d'une plage de vitesse prédéterminée à l'aide du quatrième spectre de puissance (840) estimé par l'unité d'estimation (318).

2. Appareil de traitement d'informations (30) selon la revendication 1, comprenant en outre :
une unité de mesure (114) configurée pour être montée sur une partie d'un corps d'une personne mesurée et pour mettre en œuvre la mesure de flux sanguin sur la personne mesurée.

3. Appareil de traitement d'informations (30) selon la revendication 2, dans lequel l'unité de mesure (114) comporte
une unité de rayonnement (100) configurée pour rayonner de la lumière vers la partie du corps de la personne mesurée,
une première unité de détection (102a) configurée pour détecter de la lumière provenant de la partie du corps de la personne mesurée et pour acquérir le premier signal de flux sanguin,
une seconde unité de détection (102b) configurée pour détecter de la lumière provenant de la partie du corps de la personne mesurée et pour acquérir le deuxième signal de flux sanguin et le troisième signal de flux sanguin, et
un dispositif de commande (104) configuré pour commander une fréquence d'échantillonnage qui décide d'une temporisation de rayonnement de l'unité de rayonnement (100) et d'une temporisation de lecture pour la lecture d'un résultat de détection de l'unité de détection (102).

4. Appareil de traitement d'informations (30) selon la revendication 3,
dans lequel l'unité de rayonnement (100) rayonne de la lumière avec une fréquence prédéterminée, et
l'unité de détection (102) détecte de la lumière d'interférence entre de la lumière avec la fréquence prédéterminée et de la lumière avec une fréquence différente de la fréquence prédéterminée.

5. Procédé implémenté par ordinateur comprenant :
l'estimation, en fonction d'informations de relation, d'un quatrième spectre de puissance (840) correspondant à une première fréquence d'échantillonnage à partir d'un troisième spectre de puissance (830) obtenu par le biais d'une troisième mesure de flux sanguin à une seconde fréquence d'échantillonnage inférieure à la première fréquence d'échantillonnage, dans lequel les informations de relation sont des informations indiquant une relation entre un premier spectre de puissance (810) obtenu par le biais d'une première mesure de flux sanguin à la première fréquence d'échantillonnage et un deuxième spectre de puissance (820) obtenu par le biais d'une deuxième mesure de flux sanguin à la seconde fréquence d'échantillonnage ;
la génération du premier spectre de puissance (810) à partir d'un premier signal de flux sanguin obtenu par le biais de la première mesure de flux sanguin ;
la génération du troisième spectre de puissance (830) à partir d'un troisième signal de flux sanguin obtenu par le biais de la troisième mesure de flux sanguin et du deuxième spectre de puissance (820) à partir d'un deuxième signal de flux sanguin obtenu par le biais de la deuxième mesure de flux sanguin ;
la mise en œuvre d'un apprentissage automatique à l'aide du premier spectre de puissance (810) et du deuxième spectre de puissance (820) pour apprendre les informations de relation ;
le stockage des informations de relation ; et
le calcul d'une vitesse de flux sanguin ou de densités relatives de particules dans des vaisseaux sanguins au sein d'une plage de vitesse prédéterminée à l'aide du quatrième spectre de puissance (840) estimé.

6. Programme d'ordinateur comprenant des instructions qui, lorsque le programme est exécuté par un ordinateur, amènent l'ordinateur à effectuer le procédé implémenté par ordinateur selon la revendication 5.
